# EUROPÄISCHE PATENTSCHRIFT

(11) **EP 4 039 220 B1**
(45) Veröffentlichungstag und Bekanntmachung des Hinweises auf die Patenterteilung: **28.12.2022**
(21) Anmeldenummer: 21155529.7
(22) Anmeldetag: 05.02.2021
(51) Int. Cl.: A61K 6/61, A61K 6/887, C08F 4/40, A61C 5/70, C08F 222/10

(54) **DENTALMATERIALIEN AUF DER BASIS VON POLYMERISATIONSFÄHIGEN THIOHARNSTOFF-DERIVATEN**
DENTAL MATERIALS ON THE BASIS OF POLYMERISABLE THIOUREA DERIVATIVES
MATIÈRES DENTAIRES À BASE DE DÉRIVÉS POLYMERISABLES DE THIOUREE

(43) Veröffentlichungstag der Anmeldung: 10.08.2022
(73) Patentinhaber: Ivoclar Vivadent AG, 9494 Schaan (LI)
(72) Erfinder: MOSZNER, Norbert, 9495 Triesen (LI); CATEL, Yohann, 9475 Sevelen (CH); LAMPARTH, Iris, 9472 Grabs (CH); ANGERMANN, Jörg, 7320 Sargans (CH)
(74) Vertreter: Uexküll & Stolberg

(56) Entgegenhaltungen:
- WO-A1-2007/016508
- WO-A2-03/057792

## Beschreibung

Die vorliegende Erfindung betrifft radikalisch polymerisierbare Zusammensetzungen mit einem Hydroperoxid-Thioharnstoff-Redoxinitiatorsystem, das radikalisch polymerisierbare-Thioharnstoffderivate enthält. Die Zusammensetzungen eignen sich besonders als Dentalmaterialien, beispielsweise als Prothesenmaterialien, Zemente, Adhäsive und Komposite für direkte Füllungen.

Die Haupteinsatzgebiete von Polymeren im Dentalbereich sind die abnehmbare (z.B. Zähne und Prothesenbasismaterialien) und festsitzende Prothetik (z.B. Verblendmaterialen, Kronen oder Zemente), Füllungsmaterialien (z.B. direkte oder indirekte Füllungskomposite, Befestigungszemente oder Adhäsive) oder Hilfsmaterialien (z.B. Abformmaterialien). Die Polymeren werden gewöhnlich durch radikalische Polymerisation von Zusammensetzungen erhalten, die eine polymerisierbare organische Matrix enthalten, in der Regel eine Mischung von Monomeren, Initiator-Komponenten und Stabilisatoren.

Als Monomere werden meistens Methylmethacrylat (MMA) (Prothesenmaterialien), Mischungen von funktionalisierten Monomeren, wie z.B. 2-Hydroxyethylmethacrylat (HEMA), oder säuregruppenhaltigen Haftmonomeren, wie z.B. 10-Methacryloyloxydecyldihydrogenphosphat (MDP), mit Dimethacrylaten (Adhäsive) oder Mischungen eingesetzt, die ausschließlich Dimethacrylate enthalten (Kompositzemente und Füllungskomposite). Häufig verwendete Dimethacrylate sind 2,2-Bis[4-(2-hydroxy-3-methacryloyloxypropyl)phenyl]propan (Bis-GMA) und 1,6-Bis-[2-methacryloyloxy-ethoxycarbonylamino]-2,2,4-trimethylhexan (UDMA), die eine hohe Viskosität haben und zu Polymerisaten mit sehr guten mechanischen Eigenschaften führen. Als reaktive Verdünner finden vor allem Triethylenglycoldimethacrylat (TEGDMA), 1,10-Decandioldimethacrylat (D3MA) oder Bis(3-methacryloyloxymethyl)tricyclo-[5.2.1.02.6]-decan (DCP) Anwendung.

Die Aushärtung von methacrylatbasierenden Dentalmaterialien erfolgt durch radikalische Polymerisation, wobei je nach Anwendungsgebiet Photoinitiatoren (Lichthärtung, direkte Füllungskomposite und Adhäsive), thermische Initiatoren (indirekte Komposite oder Prothesenmaterialien) oder Redox-Initiatorsysteme (Kompositzemente) eingesetzt werden. Üblich ist auch die Kombination von Photoinitiatoren mit Redoxinitiatoren, z.B. bei Füllungsmaterialien für tiefe Kavitäten.

Redoxsysteme kommen vor allem dann zum Einsatz, wenn z.B. bei Prothesenmaterialien wegen einer geringen Monomerreaktivität oder bei Befestigungszementen aufgrund unzureichender Durchstrahlung eine unvollständige Aushärtung zu befürchten ist.

Um eine ausreichende Lagerstabilität der Materialien zu gewährleisten, werden Werkstoffe auf der Basis von Redoxinitiatoren meist als sogenannte ZweiKomponenten-Systeme (2K-Systeme) eingesetzt, wobei das Oxydationsmittel (Peroxid- oder Hydroperoxid) und das Reduktionsmittel (Amine, Sulfinsäuren, Barbiturate, Thioharnstoff etc.) in zwei getrennte Komponenten eingearbeitet werden. Diese Komponenten werden kurz vor der Anwendung miteinander gemischt. Dabei müssen die beiden Komponenten so abgestimmt sein, dass ihre homogene Vermischung und Anwendung einfach möglich ist und dass eine für dentale Zwecke ausreichende Verarbeitungszeit erreicht wird. Unter der Verarbeitungszeit wird die Zeitspanne zwischen dem Vermischen der beiden Komponenten und der beginnenden Aushärtung des gemischten Materials verstanden. Andererseits darf die Aushärtungszeit, d.h. der Zeitraum bis zur vollständigen Härtung der Materialen, nicht zu lang sein.

Für dentale Kompositzemente wurden für lange Zeit vor allem Redoxinitiatorsysteme eingesetzt, die auf Mischungen von Dibenzoylperoxid (DBPO) mit tertiären aromatischen Aminen, wie z.B. N,N-Diethanol-p-toluidin (DEPT), N,N-Dimethyl-sym.-xylidin (DMSX) oder N,N-Diethyl-3,5-di-tert.-butylanilin (DABA) beruhen. Mit DBPO/Aminbasierenden Redoxinitiatorsystemen lassen sich die Verarbeitungs- und Aushärtezeit in Kombination mit phenolischen Inhibitoren relativ gut einstellen. Ein Nachteil solcher DBPO/Amin-Systeme sind Verfärbungen, die durch eine langsame Oxidation der Amine verursacht werden. Außerdem wird die Radikalbildung bei DBPO/Aminbasierenden Redoxinitiatorsystemen durch Säuren und damit auch durch saure Monomere beeinträchtigt, die regelmäßig zur Herstellung von Schmelz-Dentin-Adhäsiven eingesetzt werden. Die Aminkomponente wird durch eine Säure-Base-Reaktion protoniert und dadurch deaktiviert.

Die voranstehenden Nachteile können mit Hydroperoxid-Redoxinitiatorsystemen zum Teil überwunden werden, weil keine tertiären Amine als Reduktionsmittel erforderlich sind. Außerdem sind Hydroperoxide thermisch stabiler als Peroxide. Cumolhydroperoxid weist beispielsweise eine 10-Stunden-Halbwertstemperatur T_{1/2} von 158 °C auf, die 10-Stunden-Halbwertstemperatur T_{1/2} von DBPO beträgt nur 73 °C.

Die DE 26 35 595 C2 offenbart polymerisierbare Zahnfüllmassen, die als Initiatorsystem ein substituiertes Thioharnstoff-Reduktionsmittel in Kombination mit einem Hydroperoxid-Oxydationsmittel enthalten. Die Materialien sollen eine verbesserte Farbbeständigkeit und Lagerfähigkeit und eine gute Härtegeschwindigkeit aufweisen.

Die EP 1 693 046 B1 offenbart dentale Zemente und Stumpfaufbaumaterialien, die ein (2-Pyridyl)-2-thioharnstoffderivat in Kombination mit einem Hydroperoxid enthalten, in dem die Hydroperoxidgruppe an ein tertiäres Kohlenstoffatom gebunden ist.

Die WO 2007/016508 A1 offenbart polymerisierbare dentale Zusammensetzungen, die als Initiatorsystem ein Thioharnstoffderivat in Kombination mit einem Hydroperoxid enthalten. Die Zusammensetzungen enthalten keine Monomeren mit Säuregruppen.

Gemäß EP 1 754 465 B1 lässt sich die Reaktivität des Cumolhydroperoxid/Acetylthioharnstoff-Systems durch die Zugabe von löslichen Kupferverbindungen steigern.

Die US 7,275,932 B2 schlägt die Verwendung von Hydroperoxiden und Thioharnstoffderivaten in Kombination mit sauren Verbindungen als Beschleuniger vor. Bevorzugte saure Verbindungen sind Acrylate und Methacrylate mit Säuregruppen wie z.B. Methacrylsäure.

Die EP 2 233 544 A1 und die EP 2 258 336 A1 offenbaren Dentalwerkstoffe, die ein Hydroperoxid und ein Thioharnstoffderivat in Kombination mit einer Vanadiumverbindung als Beschleuniger enthalten.

Die WO 03/057792 A2 offenbart Dentalmaterialien, die polymerisationsfähige Thioharnstoff-Derivate wie Allylthioharnstoff, 1-Allyl-3-(2-hydroxyethyl)-2-thioharnstoff oder Methacrylsäure-4-oxo-9-thioxo-5-oxa-3,8,10-triazatridec-12-en-1-ylester als Reduktionsmittel enthalten. Diese sollen ein geringeres Risiko für potentielle toxische oder narkotisierende Nebenwirkungen aufweisen.

Initiatorsysteme auf der Basis von Hydroperoxiden und Thioharnstoffderivaten haben eine erhebliche Bedeutung bei der Vermeidung der mit Peroxid/Aminsystemen verbundenen Nachteile erlangt. Nachteilig ist, dass viele Thioharnstoffderivate einen intensiven bitteren Geschmack aufweisen (D. Mela, Chem. Senses 14 (1989) 131-135; Lange et al., Chem. Amer. Chem. Soc. 51 (1929) 1911-1914; Qin et al., Talanta 199 (2019) 131-139), der auch nach der Härtung noch wahrnehmbar ist, was von vielen Patienten als unangenehm empfunden wird.

Der Erfindung liegt die Aufgabe zugrunde, Dentalmaterialien zur Verfügung zu stellen, die nicht die Nachteile des Standes der Technik aufweisen. Die Materialien sollen nicht bitter schmecken, über eine gute Biokompatibilität und eine gute Aushärtungscharakteristik verfügen und gute mechanische Eigenschaften haben.

Diese Aufgabe wird durch radikalisch polymerisierbare Dentalwerkstoffe gelöst, die als Initiatorsystem für die radikalische Polymerisation eine Kombination aus einem Hydroperoxid und einem Thioharnstoffderivat gemäß der folgenden Formel (I) enthalten: 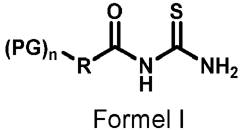 in der die Variablen die folgenden Bedeutungen haben:
- R: entfällt oder ein (n+1)-wertiger, aromatischer, aliphatischer, linearer oder verzweigter C₁-C₅₀-Kohlenwasserstoffrest, der durch eine oder mehrere, vorzugsweise 1 bis 8, besonders bevorzugt 1 bis 6, Ether-, Thioether-, Ester-, Amid- oder Urethangruppen unterbrochen sein kann,
- PG: eine radikalisch polymerisierbare (Meth)acrylat-, (Meth)acrylamid- oder Vinyl-Gruppe,
- n: ist 1, 2 oder 3.

Vorzugsweise haben die Variablen die folgenden Bedeutungen:
- R: ein (n+1)-wertiger, aromatischer, aliphatischer, linearer oder verzweigter C₁-C₃₀-Kohlenwasserstoffrest, der durch ein oder mehrere, vorzugsweise 1 bis 6, besonders bevorzugt 1 bis 3, Ether-, Ester- oder Urethangruppen unterbrochen sein kann,
- PG: eine radikalisch polymerisierbare Methacrylat- oder Vinyl-Gruppe und
- n: ist 1 oder 2.

Besonders bevorzugt haben die Variablen die folgenden Bedeutungen:
- R: ein (n+1)-wertiger aliphatischer, linearer oder verzweigter C₁-C₂₀-Kohlenwasserstoffrest, der durch 1 bis 6 Ethergruppen oder eine (1) Ester- oder Urethangruppe unterbrochen sein kann,
- PG: eine radikalisch polymerisierbare Methacrylat- oder Vinyl-Gruppe und
- n: ist 1
oder
- R: ein Phenylenrest, vorzugsweise eine p-Phenylenrest, oder ein Rest mit der Formel -Ph-CH₂-,
- PG: eine Vinyl-Gruppe und
- n: ist 1, wobei dann, wenn R -Ph-CH₂- ist, die Vinyl-Gruppe an den Phenylrest (Ph) gebunden ist: H₂C=CH-Ph-CH₂-.

Alle hierin gezeigten Formeln erstrecken sich nur auf solche Verbindungen, die mit der chemischen Valenzlehre vereinbar sind. Der Hinweis, dass ein Rest z.B. durch ein oder mehrere Ethergruppen unterbrochen ist, ist so zu verstehen, dass diese Gruppen jeweils in die Kohlenstoffkette des Restes eingeschoben werden. Diese Gruppen sind damit beidseitig durch C-Atome begrenzt und können nicht endständig sein. C₁-Reste können nicht unterbrochen sein. Unter aromatischen Kohlenwasserstoffresten werden der üblichen Nomenklatur entsprechend auch solche Reste verstanden, die aromatische und nicht aromatische Gruppen enthalten. Ein bevorzugter aromatischer Rest ist beispielsweise -Ph-CH₂-.

Die für die einzelnen Variablen angegebenen bevorzugten, besonders bevorzugten und ganz besonders bevorzugten Definitionen können jeweils unabhängig voneinander ausgewählt werden. Verbindungen, in denen alle Variablen die bevorzugten, besonders bevorzugten und ganz besonders bevorzugten Definitionen aufweisen, sind naturgemäß erfindungsgemäß besonders geeignet.

Thioharnstoff-Derivate der Formel I sind nicht bekannt, lassen sich aber nach bekannten Synthesemethoden herstellen. Beispielsweise lassen sich polymerisationsfähige Benzoyl- und Acetylhioharnstoff-Derivate durch Umsetzung der entsprechenden polymerisationsfähigen Säurechloride mit Thioharnstoff bei höheren Temperaturen herstellen:

### 1. Stufe:

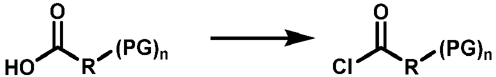

### Konkrete Beispiele:

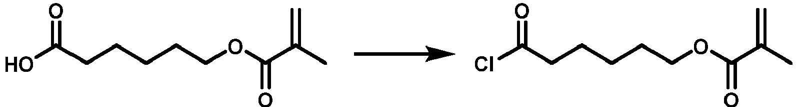

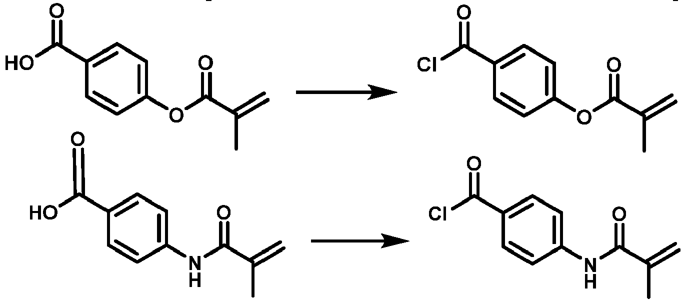

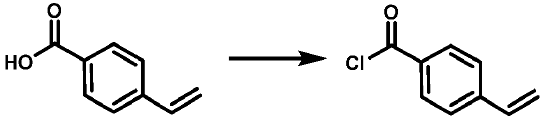

### 2. Stufe:

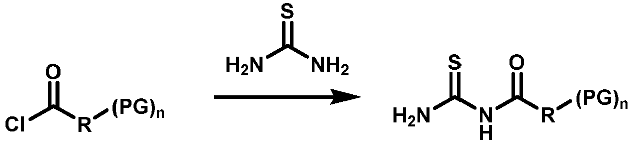

### Konkrete Beispiele:

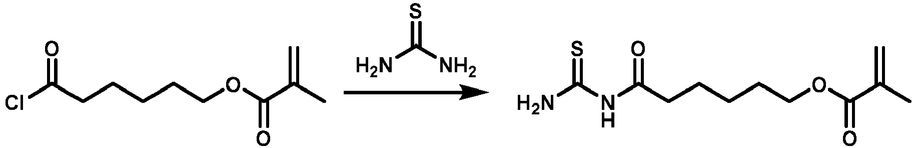

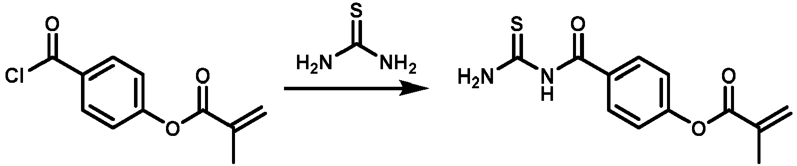

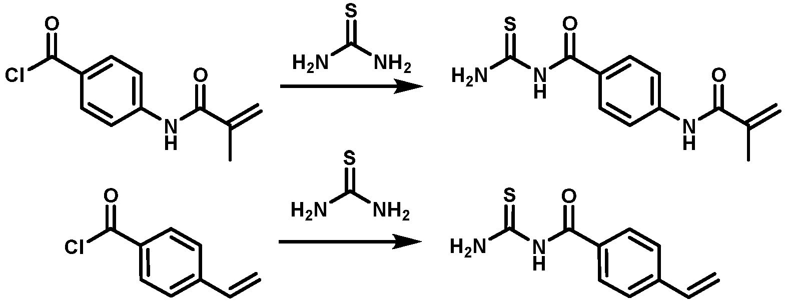

Erfindungsgemäß bevorzugte polymerisierbaren Thioharnstoff-Derivate der Formel (I) sind:

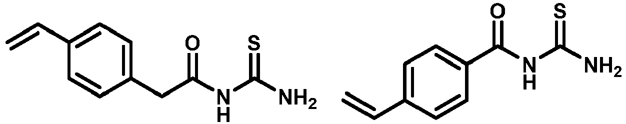

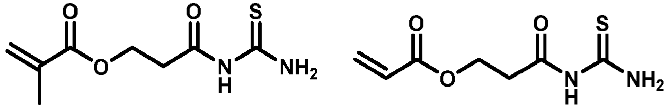

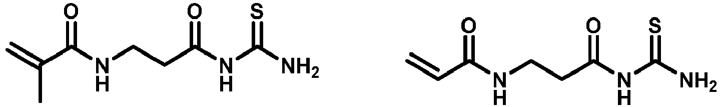

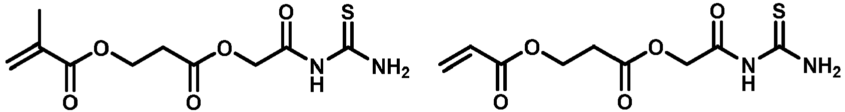

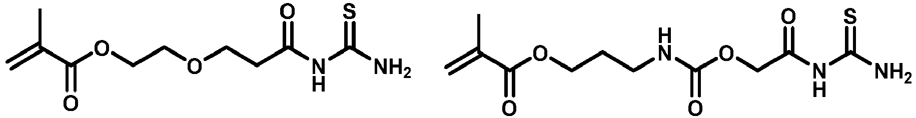

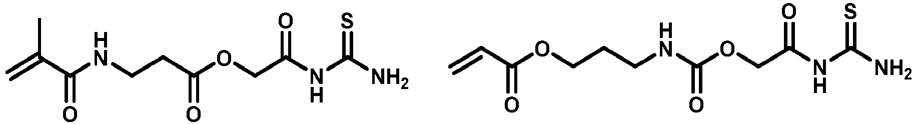

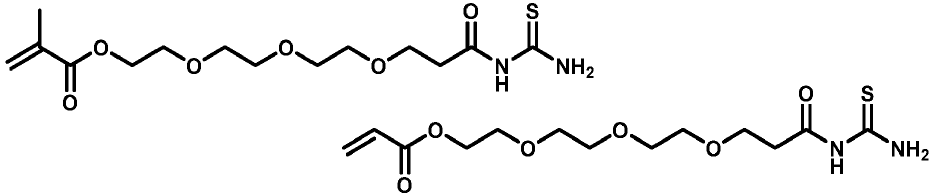

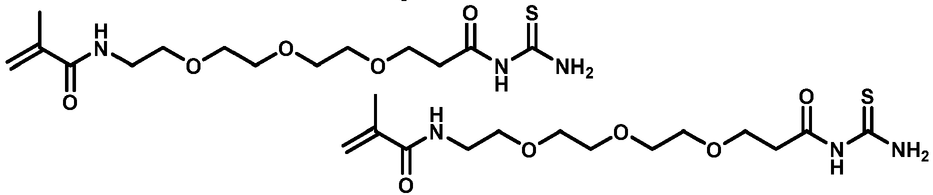

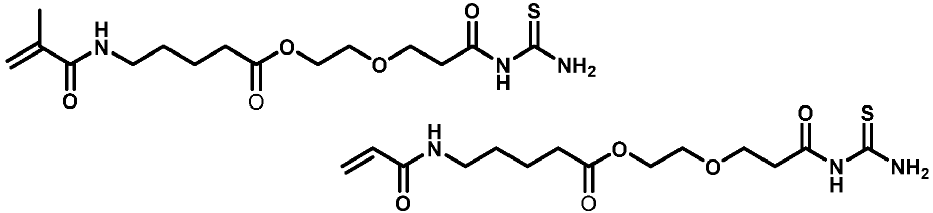

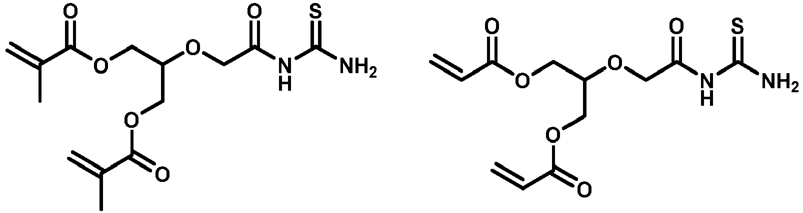

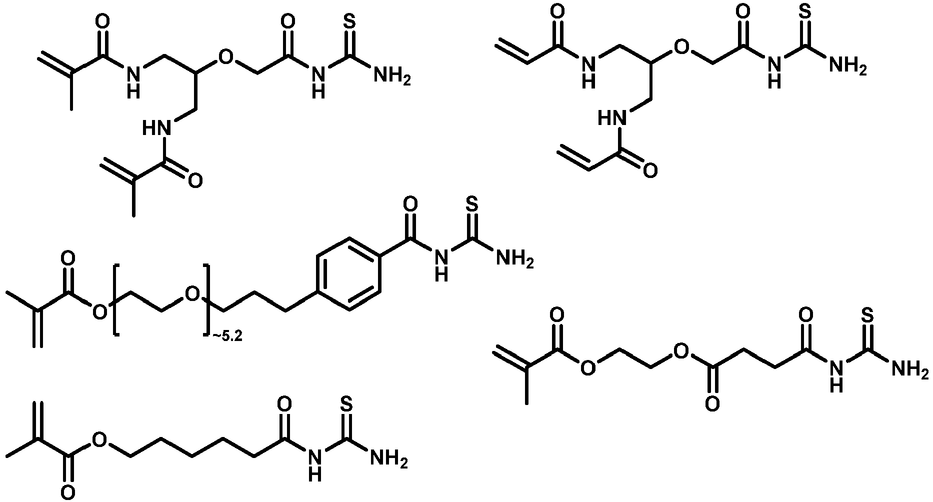

Die erfindungsgemäßen polymerisierbaren Thioharnstoff-Derivate der Formel (I) werden in Kombination mit einem Hydroperoxid eingesetzt.

Erfindungsgemäß bevorzugte Hydroperoxide sind Verbindungen der Formel R¹-(OOH)ₘ, in der R¹ ein aliphatischer oder aromatischer Kohlenwasserstoffrest und m 1 oder 2 ist. Bevorzugte Reste R¹ sind Alkyl- und Arylgruppen. Die Alkylgruppen können geradkettig, verzweigt oder cyclisch sein. Cyclische Alkylreste können durch aliphatische Alkylgruppen substituiert sein. Bevorzugt sind Alkylgruppen mit 4 bis 10 Kohlenstoffatomen. Arylgruppen können unsubstituiert oder durch Alkylgruppen substituiert sein. Bevorzugte aromatische Kohlenwasserstoffreste sind Benzolreste, die mit 1 oder 2 Alkylgruppen substituiert sind. Die aromatischen Kohlenwasserstoffreste enthalten vorzugsweise 6 bis 12 Kohlenstoffatome. Besonders bevorzugte Hydroperoxide sind t-Amylhydroperoxid, 1,1,3,3-Tetramethylbutylhydroperoxid, t-Butylhydroperoxid, t-Hexylhydroperoxid, 2,5-Dimethyl-2,5-di(hydroperoxy)hexan, Diisopropylbenzolmonohydroperoxid, Paramenthanhydroperoxid, p-Isopropylcumolhydroperoxid und Mischungen davon. Ganz besonders bevorzugt ist Cumolhydroperoxid (CHP).

Weiter bevorzugt sind die in der europäischen Patentanmeldung EP 3 692 976 A1 offenbarten geruchsarmen CHP-Derivate der Formel II 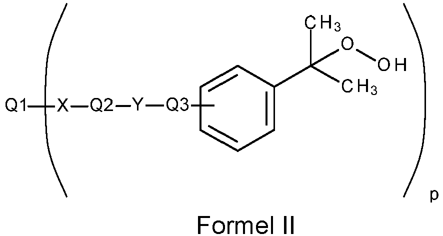 in der die Variablen die folgenden Bedeutungen haben:
- Q¹: ein p-wertiger, aromatischer, aliphatischer, linearer oder verzweigter C₁-C₁₄-Kohlenwasserstoffrest, der durch ein oder mehrere S- und/oder O-Atome unterbrochen sein kann und der unsubstituiert oder durch einen oder mehrere Substituenten, die vorzugsweise aus -OH, -OR², -Cl und -Br ausgewählt sind, substituiert sein kann, wobei R² ein aliphatischer, linearer oder verzweigter C₁-C₁₀-Kohlenwassrstoffrest ist,
- X, Y: unabhängig voneinander jeweils entfällt, -O-, -COO-; -CONR³-, oder -O-CO-NR⁴-, wobei R³ und R⁴ unabhängig voneinander für H oder einen C₁-C₅-Alkylrest, vorzugsweise für H, Methyl und/oder Ethyl, besonders bevorzugt H stehen, und wobei X und Y vorzugsweise nicht gleichzeitig entfallen,
- Q²: entfällt, ein aliphatischer, linearer oder verzweigter C₁-C₁₄-Alkylen-Rest, der durch S- und/oder O-Atome unterbrochen sein kann und der unsubstituiert oder durch -OH, -OR⁵, -Cl und/oder -Br substituiert sein kann, wobei R⁵ ein aliphatischer, linearer oder verzweigter C₁-C₁₀-Kohlenwasserstoffrest ist,
- Q³: eine C₁-C₃₋Alkylengruppe oder entfällt, vorzugsweise -CH₂- oder entfällt, wobei X und/oder Y entfällt, wenn Q² entfällt,
- p: 1, 2, 3 oder 4, und wobei
die Substitution am Aromaten in 2-, 3- oder 4-Stellung erfolgt, relativ zur Cumolhydroperoxidgruppe.

Vorzugsweise haben die Variablen die folgenden Bedeutungen:
- Q¹: ein 1- oder 2-wertiger, aliphatischer, linearer oder verzweigter C₁-C₁₀-Kohlenwasserstoffrest, der durch ein oder mehrere O-Atome, vorzugsweise ein O-Atom, unterbrochen sein kann und der durch einen oder mehrere, vorzugsweise einen, Substituenten, die aus -OH und -OR² ausgewählt sind, substituiert sein kann oder vorzugsweise unsubstituiert ist, wobei R² ein aliphatischer, linearer oder verzweigter C₁-C₆-Kohlenwassrstoffrest ist,
- X, Y: unabhängig voneinander jeweils entfällt, -O-, -COO- oder -O-CO-NR⁴-, wobei R⁴ für H oder einen C₁-C₅-Alkylrest, vorzugsweise für H, Methyl und/oder Ethyl und ganz besonders bevorzugt für H steht, und wobei X und Y vorzugsweise nicht gleichzeitig entfallen,
- Q²: entfällt, ein linearer oder verzweigter C₁-C₁₀-Alkylen-Rest, der durch ein oder mehrere O-Atome, vorzugsweise ein O-Atom, unterbrochen sein kann und der durch einen oder mehrere, vorzugsweise einen, Substituenten, die aus -OH und -OR⁵ ausgewählt sind, substituiert sein kann oder vorzugsweise unsubstituiert ist, wobei R⁵ ein aliphatischer, linearer oder verzweigter C₁-C₆-Kohlenwassrstoffrest ist,
- p: 1 oder 2, und wobei
die Substitution am Aromaten in 3-, vorzugsweise in 4-Stellung erfolgt.

Besonders bevorzugt haben die Variablen die folgenden Bedeutungen:
- Q¹: ein 1- oder 2-wertiger, aliphatischer, linearer oder verzweigter C₁-C₅-Kohlenwasserstoffrest, der durch ein O-Atom unterbrochen sein kann und der durch eine OH-Gruppe substituiert sein kann,
- X: -COO-,
- Y: entfällt,
- Q²: entfällt oder ein linearer C₁-C₃-Alkylen-Rest,
- p: 1 oder 2, und wobei die Substitution am Aromaten in 4-Stellung erfolgt.

Ganz besonders bevorzugt haben die Variablen die folgenden Bedeutungen:
- Q¹: ein 1- oder 2-wertiger, aliphatischer, verzweigter, vorzugsweise linearer C₁-C₄-Kohlenwasserstoffrest,
- X: -COO-,
- Y: entfällt,
- Q²: entfällt oder ein Methylen-Rest,
- p: 1 oder 2, und wobei die Substitution am Aromaten in 4-Stellung erfolgt.

Erfindungsgemäß bevorzugte Hydroperoxid-Derivate der Formel II sind:

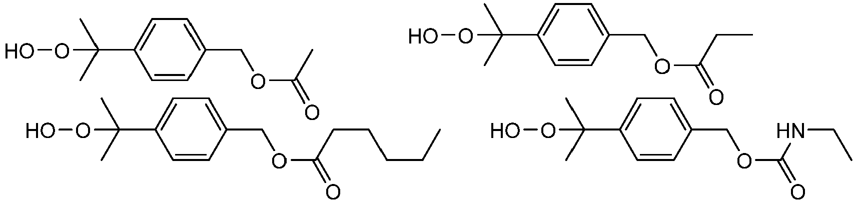

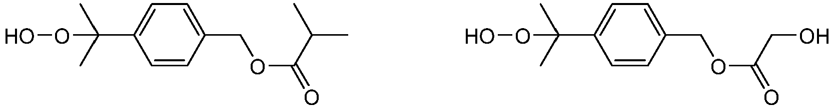

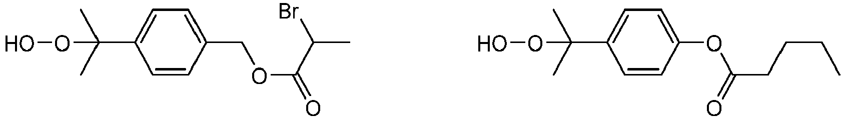

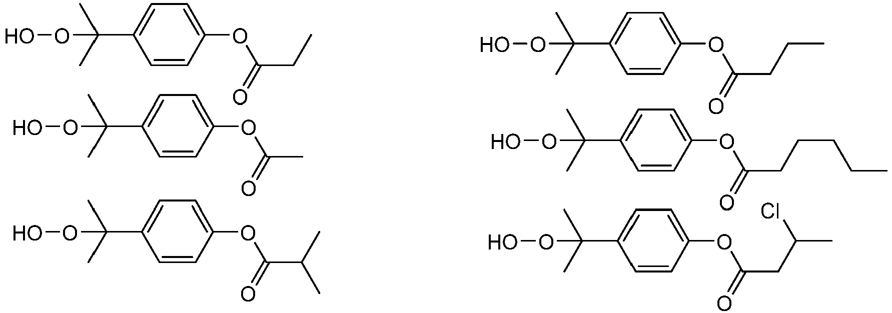

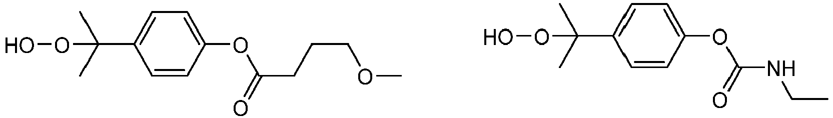

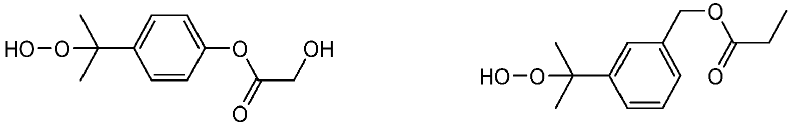

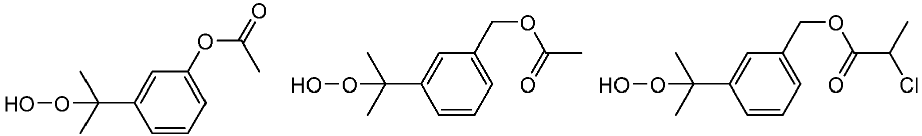

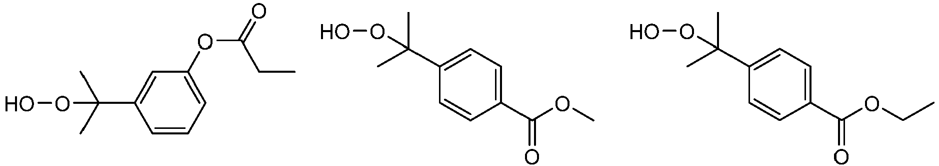

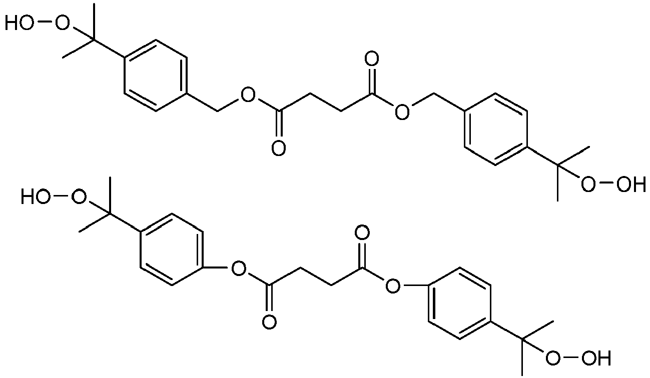

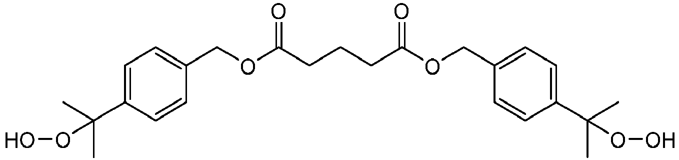

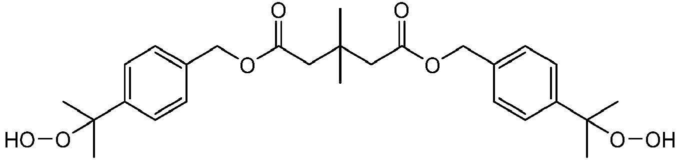

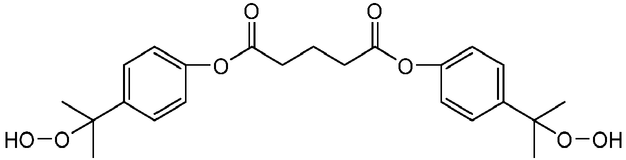

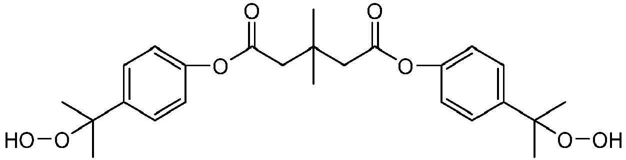

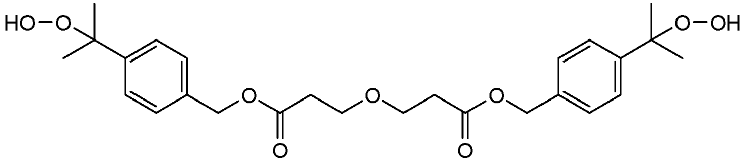

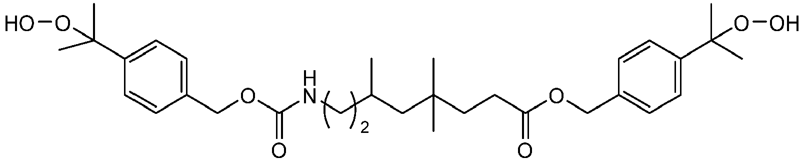

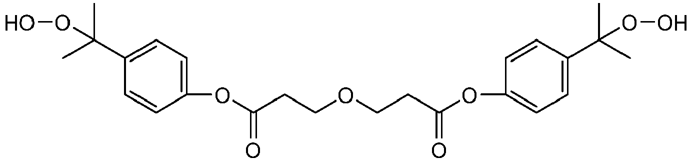

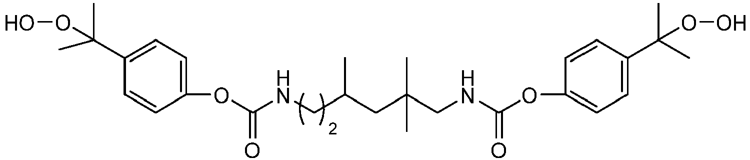

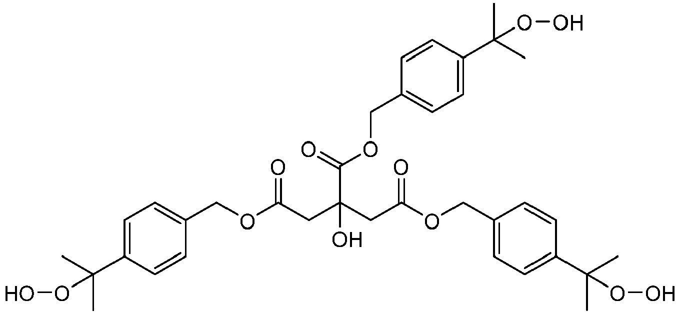

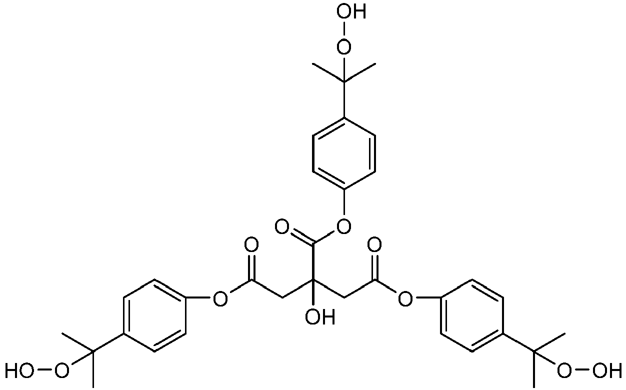

Die Hydroperoxid-Derivate der Formel II zeigen eine große Lagerstabilität bei Raumtemperatur und eignen sich besonders als geruchsarme Hydroperoxid-Komponente in Redoxinitiatorsystemen für dentale Zusammensetzungen. Die erfindungsgemäßen Werkstoffe können ein oder mehrere Hydroperoxide enthalten.

Gemäß einer weiteren bevorzugten Ausführungsform enthalten die erfindungsgemäßen Dentalwerkstoffe neben mindestens einem Hydroperoxid und mindestens einem Thioharnstoffderivat der Formel I zusätzlich mindestens eine Übergangsmetallverbindung. Es wurde gefunden, dass die Zugabe einer Übergangsmetallverbindung Werkstoffe ergibt, die nach der Härtung erheblich verbesserte mechanische Eigenschaften aufweisen.

Erfindungsgemäß bevorzugte Übergangsmetallverbindungen sind Verbindungen, die sich von Übergangsmetallen ableiten, die mindestens zwei stabile Oxidationsstufen haben. Besonders bevorzugt sind Verbindungen der Elemente Kupfer, Eisen, Kobalt, Nickel und Mangan. Diese Metalle weisen die folgenden stabilen Oxydationsstufen auf: Cu(I)/Cu(II), Fe(II)/Fe(III), Co(II)/Co(III), Ni(II)/Ni(III), Mn(II)/Mn(III). Werkstoffe, die mindestens eine Kupfer-Verbindung enthalten, sind besonders bevorzugt.

Die Übergangsmetalle werden bevorzugt in Form ihrer Salze eingesetzt. Bevorzugte Salze sind die Nitrate, Acetate, 2-Ethyl-hexanoate und Halogenide, wobei Chloride besonders bevorzugt sind.

Die Übergangsmetalle können weiterhin vorteilhaft in komplexierter Form eingesetzt werden, wobei Komplexe mit chelatbildenden Liganden besonders bevorzugt sind. Bevorzugte einfache Liganden zur Komplexierung der Übergangsmetalle sind 2-Ethylhexanoat und THF. Bevorzugte chelatbildende Liganden sind 2-(2-Aminoethyl-amino)ethanol, aliphatische Amine, besonders bevorzugt 1,1,4,7,10,10-Hexamethyltriethylentetramin (HMTETA), N,N,N',N",N"-Pentamethyldiethylentriamin (PMDETA), Tris[2-(dimethylamino)ethyl]amin (Me₆TREN), N,N,N',N'-Tetramethylethylendiamin (TMEDA), 1,4,8,11-Tetraaza-1,4,8,11-tetramethylcyclotetradecan (Me4CYCLAM), Diethylentriamin (DETA), Triethylentetramin (TETA) und 1,4,8,11-Tetraazacyclotetradecan (CYCLAM)); pyridinhaltige Liganden, besonders bevorzugt N,N,N',N'-Tetrakis-(2-pyridylmethyl)ethylendiamin (TPEN), N,N-Bis(2-pyridylmethyl)amin (BPMA), N,N-Bis(2-pyridylmethyl)octylamin (BPMOA), 2,2'-Bipyridin und 8-Hydroxychinolin. Ganz besonders bevorzugte Liganden sind Acetylaceton, Dimethylglyoxim und 1,10-Phenanthrolin.

Bei elektrisch neutralen Liganden muss die Ladung der Übergangsmetallionen durch geeignete Gegenionen ausgeglichen werden. Hierzu kommen insbesondere die oben genannten Ionen in Betracht, die zur Bildung von Salzen verwendet werden, wobei Acetate und Chloride besonders bevorzugt sind. Chloride und Komplexe zeichnen sich durch eine relativ gute Löslichkeit in Monomeren aus, die zur Herstellung von Dentalwerkstoffen eingesetzt werden.

Anstelle der Übergangsmetallkomplexe können nichtkomplexe Salze der Übergangsmetalle in Kombination mit komplexbildenden organischen Verbindungen zur Herstellung der Dentalwerkstoffe eingesetzt werden, vorzugsweise in Kombination mit den oben genannten chelatbildenden Verbindungen. Die organischen Liganden bilden beim Mischen mit den Übergangsmetallsalzen die katalytisch wirksamen Komplexe. Die Verwendung solcher Kombinationen von Übergangsmetallsalzen und organischen Liganden ist bevorzugt.

Bevorzugt sind Übergangsmetallverbindungen der Metalle Kupfer, vorzugsweise Cu⁺, Eisen, vorzugsweise Fe³⁺, Kobalt, vorzugsweise Co³⁺, und Nickel, vorzugsweise Ni²⁺.

Bevorzugte Kupfersalze sind Cu(II)-carboxylate (z.B. der Essigsäure oder 2-Ethylhexansäure), CuCl₂, CuBr₂, CuI₂, besonders bevorzugt CuBr und ganz besonders bevorzugt CuCI. Bevorzugte Kupferkomplexe sind Komplexe mit den Liganden Acetylaceton, Phenanthrolin (z.B. 1,10-Phenanthrolin (Phen)), aliphatischen Aminen, wie z.B. 1,1,4,7,10,10-Hexamethyltriethylentetramin (HMTETA), N,N,N',N",N"-Penta-methyldiethylentriamin (PMDETA), Tris[2-(dimethylamino)ethyl]amin (Me₆TREN).

Bevorzugte Eisensalze sind FeCl₃, FeBr₂ und FeCl₂. Bevorzugte Eisenkomplexe sind Komplexe mit den Liganden Acetylaceton, Triphenylphosphin, 4,4'-Di(5-nonyl)-2,2'-bipyridin (dNbpy) oder 1,3-Diisopropyl-4,5-dimethylimidazol-2-yliden (Prilm). Ganz besonders bevorzugt sind die Komplexe Fe(acac)₂ und FeCl₂(PPh₃)₂.

Bevorzugte Nickelsalze sind NiBr₂ und NiCl₂, bevorzugte Nickelkomplexe sind Nickelacetylacetonat und NiBr₂(PPh₃)₂.

Erfindungsgemäß sind Kupferverbindungen, Kupferkomplexe und insbesondere Mischungen von Kupfersalzen und komplexierenden organischen Liganden besonders bevorzugt. Ganz besonders bevorzugt sind Salze und Komplexe des einwertigen Kupfers (Cu⁺), am meisten bevorzugt ist Kupfer(I)chlorid (CuCI). Zusammensetzungen, die ein Salz des einwertigen Kupfers und insbesondere CuCI enthalten, zeichnen sich durch eine besonders gute Lagerstabilität aus.

Die erfindungsgemäßen Werkstoffe enthalten vorzugsweise 0,001 bis 5 Gew.-%, besonders bevorzugt 0,005 bis 3,0 Gew.-% und ganz besonders bevorzugt 0,1 bis 3,0 Gew.-% mindestens eines Thioharnstoffderivats der Formel (I).

Das oder die Hydroperoxide werden vorzugsweise in einer (Gesamt-)Menge von 0,01 bis 5 Gew.-%, besonders bevorzugt 0,05 bis 4,0 Gew.-% und ganz besonders bevorzugt 0,1 bis 3,0 Gew.-% eingesetzt.

Die Übergangsmetallverbindung wird ggf. vorzugsweise in einer Menge von 0,0001 bis 1 Gew.-%, bevorzugt 0,0005 bis 0,5 Gew.-% und besonders bevorzugt 0,0007 bis 0,020 Gew.-% eingesetzt.

Wenn nicht anders angegeben beziehen sich alle Prozentangaben hierin auf die Gesamtmasse der Zusammensetzung.

Thioharnstoffderivate der Formel (I) eignen sich in Kombination mit mindestens einem Hydroperoxid besonders zur Aushärtung radikalisch polymerisierbarer Zusammensetzungen.

Die erfindungsgemäßen Werkstoffe enthalten vorzugsweise mindestens ein radikalisch polymerisierbares Monomer. Besonders bevorzugt sind Zusammensetzungen, die als radikalisch polymerisierbares Monomer mindestens ein mono- oder multifunktionelles (Meth)acrylat enthalten. Unter monofunktionellen (Meth)acrylaten werden Verbindungen mit einer, unter multifunktionellen (Meth)acrylaten Verbindungen mit zwei oder mehr, vorzugsweise 2 bis 4 radikalisch polymerisierbaren Gruppen verstanden. Gemäß einer ganz besonders bevorzugten Ausführungsform enthalten die erfindungsgemäßen Zusammensetzungen mindestens ein Dimethacrylat oder eine Mischung von Mono- und Dimethacrylaten. Materialien, die intraoral gehärtet werden sollen, enthalten als radikalisch polymerisierbares Monomer vorzugsweise mono- und/oder multifunktionelle Methacrylate.

Bevorzugte mono- oder multifunktionelle (Meth)acrylate sind Methyl-, Ethyl-, 2-Hydroxyethyl-, Butyl-, Benzyl-, Tetrahydrofurfuryl- oder Isobornyl(meth)acrylat, p-Cumylphenoxyethylenglycolmethacrylat (CMP-1E), 2-(2-Biphenyloxy)-ethylmethacrylat, Bisphenol-A-dimethacrylat, Bis-GMA (ein Additionsprodukt aus Methacrylsäure und Bisphenol-A-diglycidylether), ethoxy- oder propoxyliertes Bisphenol-A-Dimethacrylat, wie z.B. 2-[4-(2-Methacryloyloxyethoxyethoxy)phenyl]-2-[4-(2-meth-acryloyloxyethoxy)phenyl]propan) (SR-348c, Firma Sartomer; enthält 3 Ethoxygruppen) und 2,2-Bis[4-(2-methacryloxypropoxy)phenyl]propan, UDMA (ein Additionsprodukt aus 2-Hydroxyethylmethacrylat und 2,2,4-Trimethylhexamethylen-1,6-diisocyanat), V-380 (ein Additionsprodukt aus einer Mischung von 0,7 mol 2-Hydroxyethylmethacrylat und 0,3 mol 2-Hydroxypropylmethacrylat mit 1 mol α,α,α',α'-Tetramethyl-m-xylylen-diisocyanat), Di-, Tri- oder Tetraethylenglycoldimethacrylat, Trimethylolpropantrimethacrylat, Pentaerythrittetramethacrylat sowie Glycerindi- und -trimethacrylat, 1,4-Butandioldimethacrylat, 1,10-Decandioldimethacrylat (D₃MA), Bis(methacryloyloxymethyl)tricyclo-[5.2.1.02,6]decan (DCP), Polyethylenglycol- oder Polypropylenglycoldimethacrylate, wie z.B. Polyethylenglycol-200- oder Polyethylenglycol-400-dimethacrylat (PEG-200- oder PEG-400-DMA) oder 1,12-Dodecandioldimethacrylat, oder eine Mischung davon.

Gemäß einer Ausführungsform enthalten die erfindungsgemäßen Zusammensetzungen neben den oben genannten Monomeren vorzugsweise zusätzlich ein oder mehrere säuregruppenhaltige radikalisch polymerisierbare Monomere (Haftmonomere). Diese verleihen den Werkstoffen selbsthaftende und/oder selbstätzende Eigenschaften. Säuregruppenhaltige Monomere eignen sich daher besonders zur Herstellung von selbsthaftenden Dentalwerkstoffen, wie z.B. von Befestigungszementen.

Bevorzugte säuregruppenhaltige Monomere sind polymerisationsfähige Carbonsäuren, Phosphonsäuren und Phosphorsäureester sowie deren Anhydride. Bevorzugte Carbonsäuren und Carbonsäureanhydride sind 4-(Meth)acryloyloxyethyltrimellit-säureanhydrid, 10-Methacryloyloxydecylmalonsäure, N-(2-Hydroxy-3-methacryloyl-oxypropyl)-N-phenylglycin, 4-Vinylbenzoesäure. Bevorzugte Phosphorsäureester sind 2-Methacryloyloxyethyl-phenyl-hydrogenphosphat, 10-Methacryloyloxydecyldihydrogenphosphat (MDP) und Dipentaerythritpentamethacryloyloxyphosphat. Bevorzugte Phosphonsäuren sind 4-Vinylbenzylphosphonsäure, 2-[4-(Dihydroxy-phosphoryl)-2-oxa-butyl]-acrylsäure und deren Amide, Ester, wie z.B. 2-[4-(Di-hydroxyphosphoryl)-2-oxa-butyl]-acrylsäure-2,4,6-trimethylphenylester.

Besonders bevorzugte säuregruppenhaltige Monomere sind 4-Vinylbenzylphosphonsäure, 2-[4-(Dihydroxyphosphoryl)-2-oxa-butyl]-acrylsäure und deren Amide, Ester, wie z.B. 2-[4-(Dihydroxyphosphoryl)-2-oxa-butyl]-acrylsäure-2,4,6-trimethylphenyl-ester, (Meth)acrylamiddihydrogenphosphate, wie z.B. 6-Methacrylamidohexyl- oder 1,3-Bis(methacrylamido)-propan-2-yl-dihydrogenphosphat, und Mischungen davon. Diese besonders bevorzugten säuregruppenhaltigen Monomere zeichnen sich durch eine hohe Hydrolysestabilität aus.

Die erfindungsgemäßen Zusammensetzungen können neben dem erfindungsgemäßen Initiatorsystem vorteilhaft zusätzlich einen Initiator für die radikalische Photopolymerisation enthalten. Solche Zusammensetzungen sind dualhärtend, d.h. sie lassen sich sowohl chemisch als auch durch Licht härten. Bevorzugte Photoinitiatoren sind Benzophenon, Benzoin sowie deren Derivate, α-Diketone und deren Derivate, wie 9,10-Phenanthrenchinon, 1-Phenyl-propan-1,2-dion, Diacetyl und 4,4'-Dichlorbenzil. Bevorzugt werden Campherchinon (CC) und 2,2-Dimethoxy-2-phenyl-acetophenon in Kombination mit Aminen als Reduktionsmittel, wie z.B. 4-(Dimethyl-amino)-benzoesäureethylester (EDMAB), oder N,N-Dimethylaminoethylmethacrylat, verwendet.

Erfindungsgemäß sind solche Zusammensetzungen bevorzugt, die keine Amine enthalten. Besonders bevorzugt sind daher Norrish-Typ-I-Photoinitiatoren. Norrish-Typ-I-Photoinitiatoren benötigen keine Aminkomponente.

Bevorzugte Norrish-Typ-I-Photoinitiatoren sind Acyl- oder Bisacylphosphinoxide. Besonders bevorzugt sind Monoacyltrialkylgermanium-, Diacyldialkylgermanium- und Tetraacylgermanium-Verbindungen, wie z.B. Benzoyltrimethylgerman, Dibenzoyldiethylgerman, Bis(4-methoxybenzoyl)diethylgerman (Ivocerin^{®}), Tetrabenzoylgerman und Tetrakis(o-methylbenzoyl)german.

Außerdem lassen sich auch Mischungen der verschiedenen Photoinitiatoren einsetzen, wie z.B. Bis(4-methoxybenzoyl)diethylgerman bzw. Tetrakis(o-methylbenzoyl)-german in Kombination mit Campherchinon und 4-Dimethylaminobenzoesäureethylester.

Die erfindungsgemäßen Dentalwerkstoffe können vorteilhaft außerdem einen oder mehrere organische oder anorganische Füllstoffe enthalten. Bevorzugt sind partikuläre Füllstoffe. Füllstoffhaltige Zusammensetzungen eignen sich besonders als dentale Befestigungszemente oder Füllungskomposite.

Bevorzugte anorganische Füllstoffe sind Oxide, wie SiO₂, ZrO₂ und TiO₂ oder Mischoxide aus SiO₂, ZrO₂, ZnO und/oder TiO₂, nanopartikuläre oder mikrofeine Füllstoffe, wie pyrogene Kieselsäure oder Fällungskieselsäure, Glaspulver, wie Quarz-, Glaskeramik-, Borosilikat- oder röntgenopake Glaspulver, vorzugsweise Barium- oder Strontiumaluminiumsilikatgläser, und röntgenopake Füllstoffe, wie Ytterbiumtrifluorid, Tantal(V)-oxid, Bariumsulfat oder Mischoxide von SiO₂ mit Ytterbium(III)-oxid oder Tantal(V)-oxid. Weiterhin können die erfindungsgemäßen Dentalwerkstoffe faserförmige Füllstoffe, Nanofasern, Whiskers oder Mischungen davon enthalten. Gemäß einer bevorzugten Ausführungsform enthalten die erfindungsgemäßen Werkstoffe keine Fluoraluminosilikatgläser, Calciumaluminiumsilikatgläser oder andere Füllstoffe, die mit organischen Säuren im Sinne einer Säure-Base-Reaktion reagieren.

Bevorzugt weisen die Oxide eine Partikelgröße von 0,010 bis 15 µm, die nanopartikulären oder mikrofeinen Füllstoffe eine Partikelgröße von 10 bis 300 nm, die Glaspulver eine Partikelgröße von 0,01 bis 15 µm, vorzugweise von 0,2 bis 1,5 µm, und die röntgenopaken Füllstoffe eine Partikelgröße von 0,2 bis 5 µm auf.

Besonders bevorzugte Füllstoffe sind Mischoxide aus SiO₂ und ZrO₂, mit einer Partikelgröße von 10 bis 300 nm, Glaspulver mit einer Partikelgröße von 0,2 bis 1,5 µm, insbesondere röntgenopake Glaspulver z.B. von Barium- oder Strontiumaluminiumsilikatgläsern, und röntgenopake Füllstoffe mit einer Partikelgröße von 0,2 bis 5 µm, insbesondere Ytterbiumtrifluorid und/oder Mischoxide von SiO₂ mit Ytterbium(III)-oxid.

Außerdem sind als Füllstoff gemahlene Präpolymerisate oder Perlpolymerisate (Isofüller) geeignet. Diese können ausschließliche aus organischen Polymeren oder aus organischen Polymeren bestehen, die ihrerseits mit anorganischen Füllstoffen wie röntgenopakem/n Glaspulver(n) und Ytterbiumtrifluorid gefüllt sind. Zur Herstellung der gemahlenen Präpolymerisate und Perlpolymere eignen sich die oben definierten Monomere und Füllstoffe. Zusammensetzungen zur Herstellung dentaler Totalprothesen enthalten als Füllstoffe vorzugsweise ausschließlich organische Füllstoffe, besonders bevorzugt gemahlene Polymerisate oder Perlpolymerisate auf Basis von Polymethylmethacrylat (PMMA), ganz besonders bevorzugt Perlpolymerisate auf der Basis von PMMA. Gemäß einer bevorzugten Ausführungsform enthalten die erfindungsgemäßen Werkstoffe keine Polymeren mit sauren Gruppen, insbesondere keine Polymeren mit Carbonsäuregruppen.

Wenn nicht anders angegeben, handelt es sich bei allen Partikelgrößen um gewichtsmittlere Partikelgrößen, wobei die Partikelgrößenbestimmung im Bereich von 0,1 µm bis 1000 µm mittels statischer Lichtstreuung erfolgt, vorzugsweise mit einem statischen Laserstreuungs-Partikelgrößen-Analysator LA-960 (Horiba, Japan). Hierbei werden als Lichtquellen eine Laser-Diode mit einer Wellenlänge von 655 nm und eine LED mit einer Wellenlänge von 405 nm verwendet. Der Einsatz von zwei Lichtquellen mit unterschiedlichen Wellenlängen ermöglicht die Vermessung der gesamten Partikelgrößenverteilung einer Probe in nur einem Messungsdurchgang, wobei die Messung als Nassmessung durchgeführt wird. Hierzu wird eine 0,1 bis 0,5%ige wässrige Dispersion des Füllstoffs hergestellt und deren Streulicht in einer Durchflusszelle gemessen. Die Streulichtanalyse zur Berechnung von Partikelgröße und Partikelgrößenverteilung erfolgt gemäß der Mie-Theorie nach DIN/ISO 13320.

Partikelgrößen kleiner als 0,1 µm werden vorzugsweise mittels Dynamischer Lichtstreuung (DLS) bestimmt. Die Messung der Partikelgröße im Bereich von 5 nm bis 0,1 µm erfolgt vorzugsweise durch Dynamische Lichtstreuung (DLS) von wässrigen Partikeldispersionen, vorzugsweise mit einem Malvern Zetasizer Nano ZS (Malvern Instruments, Malvern UK) mit einem He-Ne-Laser mit einer Wellenlänge von 633 nm, bei einem Streuwinkel von 90° bei 25°C.

Die Lichtstreuung nimmt mit abnehmender Partikelgröße ab. Partikelgrößen kleiner als 0,1 µm können auch mittels REM- oder TEM-Spektroskopie bestimmt werden. Die Transmissionselektronenmikroskopie (TEM) wird vorzugsweise mit einem Philips CM30 TEM bei einer Beschleunigungsspannung von 300 kV durchgeführt. Für die Probenvorbereitung werden Tropfen der Partikeldispersion auf einem 50 Å dickem Kupfergitter (Maschenweite 300) aufgebracht, das mit Kohlenstoff beschichtet ist, und im Anschluss das Lösungsmittel verdampft.

Die Füllstoffe werden nach ihrer Partikelgröße unterteilt in Makrofüller und Mikrofüller, wobei Füllstoffe mit einer mittleren Partikelgröße von 0,2 bis 10 µm als Makrofüller und Füllstoffe mit einer mittlere Partikelgröße von ca. 5 bis 100 nm als Mikrofüller bezeichnet werden. Makrofüller werden z.B. durch Mahlen z.B. von Quarz, röntgenopaken Gläsern, Borosilikaten oder von Keramik gewonnen und bestehen meist aus splitterförmigen Teilen. Mikrofüller wie Mischoxide können z.B. durch hydrolytische Co-Kondensation von Metallalkoxiden hergestellt werden. Füllstoffe mit geringer Partikelgröße haben eine größere Verdickungswirkung.

Zur Verbesserung des Verbundes zwischen den Füllstoffpartikeln und der vernetzten Polymerisationsmatrix sind die Füllstoffe vorzugsweise oberflächenmodifiziert, besonders bevorzugt durch Silanisierung, ganz besonders bevorzugt durch radikalisch polymerisierbare Silane, insbesondere mit 3-Methacryloyloxypropyltrimethoxysilan. Zur Oberflächenmodifizierung von nicht-silikatischen Füllstoffen, z.B. von ZrO₂ oder TiO₂, können auch funktionalisierte saure Phosphate, wie z.B. 10-Methacryloyloxydecyldihydrogenphosphat eingesetzt werden.

Außerdem können die erfindungsgemäßen Dentalwerkstoffe ein oder mehrere weitere Additive enthalten, vor allem Stabilisatoren, Farbmittel, mikrobiozide Wirkstoffe, fluoridionenabgebende Additive, Treibmittel, optische Aufheller, Weichmacher und/oder UV-Absorber. Die erfindungsgemäßen Werkstoffe können als Additive auch ein oder mehrere organische oder anorganische Lösungsmittel enthalten. Gemäß einer bevorzugten Ausführungsform enthalten die erfindungsgemäßen Werkstoffe jedoch kein Wasser.

Erfindungsgemäß sind Dentalwerkstoffe bevorzugt, die
(a) 0,001 bis 5,0 Gew.-%, bevorzugt 0,005 bis 3,0 Gew.-%, besonders bevorzugt 0,1 bis 3,0 Gew.-% mindestens eines Thioharnstoffderivats der Formel (I),
(b) 0,01 bis 5,0 Gew.-%, bevorzugt 0,05 bis 4,0 Gew.-% und besonders bevorzugt 0,1 bis 3,0 Gew.-% mindestens eines Hydroperoxids,
(c) 5 bis 95 Gew.-%, bevorzugt 10 bis 95 Gew.-% und besonders bevorzugt 10 bis 90 Gew.-% mindestens eines radikalisch polymerisierbaren Monomers,
(d) 0 bis 80 Gew.-% Füllstoff(e), und
(e) 0,01 bis 5 Gew.-%, bevorzugt 0,1 bis 3 Gew.-% und besonders bevorzugt 0,1 bis 2 Gew.-% Additiv(e) enthalten.

Alle Mengenangaben hierin beziehen sich auf die Gesamtmasse der Zusammensetzung, wenn nicht anders angegeben.

Der Füllungsgrad richtet sich nach dem gewünschten Anwendungszweck des Werkstoffs. Füllungskomposite haben vorzugsweise einen Füllstoffgehalt von 50 bis 80 Gew.-%, besonders bevorzugt 70 bis 80 Gew.-%, und dentale Zemente von 10 bis 70 Gew.-%, besonders bevorzugt 60 bis 70 Gew.-%. Prothesenmaterialien haben vorzugsweise einen Füllstoffgehalt von 0 bis 10 Gew.-%, besonders bevorzugt 0 bis 5 Gew.-%,

### Besonders bevorzugt sind Dentalwerkstoffe, die zusätzlich

(f) 0,0001 bis 1 Gew.-%, bevorzugt 0,0005 bis 0,5 Gew.-%, besonders bevorzugt 0,0007 bis 0,02 Gew.-% mindestens einer Übergangsmetallverbindung enthalten.

Besonders bevorzugt sind solche Dentalwerkstoffe, die aus den genannten Komponenten bestehen, wobei die einzelnen Komponenten vorzugsweise jeweils aus den oben genannten bevorzugten und besonders bevorzugten Stoffen ausgewählt sind. In allen Fällen kann als jeweilige Komponente auch eine Mischung von mehreren Stoffen eingesetzt werden, also beispielsweise eine Mischung von Monomeren.

Die erfindungsgemäßen Werkstoffe liegen vorzugsweise in Form von zwei getrennten Komponenten vor, von denen die erste Komponente das Hydroperoxid (Katalysatorpaste) und die zweite Komponente das Thioharnstoffderivat (Basenpaste) enthält. Da die Komponenten vorzugweise eine pastenförmige Konsistenz haben, werden sie hierin auch als Pasten bezeichnet. Die Zusammensetzung von Katalysator- und Basenpaste unterscheiden sich im Wesentlichen dadurch, dass die Katalysatorpaste ein oder mehrere Hydroperoxide und die Basenpate ein oder mehrere Thioharnstoffderivate enthält.

Die Pasten werden zur Anwendung miteinander gemischt und hierdurch die Härtungsreaktion gestartet. Die Pasten werden vorzugsweis so abgemischt, dass Katalysator- und Basenpaste in einem Volumenverhältnis von 1:1 angewendet werden können. Nach dem Mischen der Pasten weisen die Werkstoffe die oben definierte Zusammensetzung auf.

Die erfindungsgemäßen Dentalwerkstoffe zeichnen sich insbesondere dadurch aus, dass sie nach der Härtung keinen bitteren Geschmack aufweisen. Außerdem haben sie eine gute Aushärtungscharakteristik, d.h. sie weisen eine vorteilhafte Verarbeitungszeit in Kombination mit einer vorteilhaften Aushärtungszeit auf. Weiterhin haben die Werkstoffe nach der Härtung mechanische Eigenschaften, die mit Werkstoffen auf der Basis etablierter Redoxsysteme, wie z.B. einer Mischung von Acetylthioharnstoff und Cumolhydroperoxid (CHP), ohne Einschränkungen vergleichbar sind.

Die erfindungsgemäßen Zusammensetzungen eignen sich besonders als Dentalwerkstoffe, insbesondere als dentale Zemente, Füllungskomposite und Verblendmaterialien sowie als Materialien zur Herstellung von Prothesen, künstlichen Zähnen, Inlays, Onlays, Kronen und Brücken. Die Zusammensetzungen eignen sich primär zur intraoralen Anwendung durch den Zahnarzt zur Restauration geschädigter Zähne, d.h. zur therapeutischen Anwendung, z.B. als dentale Zemente, Füllungskomposite und Verblendmaterialien. Sie können aber auch nicht-therapeutisch (extraoral) eingesetzt werden, beispielsweise bei der Herstellung oder Reparatur von Dentalrestaurationen, wie Prothesen, künstlichen Zähnen, Inlays, Onlays, Kronen und Brücken.

Die Erfindung wird im Folgenden anhand von Ausführungsbeispielen näher erläutert:

### Ausführungsbeispiele

### Beispiel 1

### Synthese von N-(2-Methacryloyloxyhexanoyl)-thiohamstoff

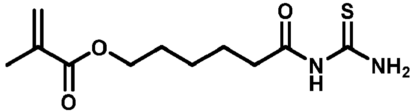

### 1. Stufe: 6-Hydroxyhexansäure-tert.-butyleste

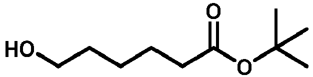

*ε*-Caprolacton (181.88 g, 1.59 mol) wurde in *tert*.-Butanol (1600 ml) gelöst. Kalium*tert*.-butoxid (196.68 g, 1.75 mol) wurde zugegeben und die Suspension wurde 5 h am Rückfluss erhitzt. Nach dem Abkühlen wurden Toluol (1600 ml) und Wasser (800 ml) zugegeben, und die Phasen wurden getrennt. Die organische Phase wurde mit Wasser (2x 1000 ml) und gesättigter wässriger Natriumchlorid-Lösung (1000 ml) gewaschen, über wasserfreiem Natriumsulfat getrocknet, filtriert und am Rotationsverdampfer eingeengt. Das Rohprodukt wurde mittel Vakuumdestillation gereinigt (Siedepunkt: 70°C/0.03 mbar). Man erhielt 56.57 g (0.30 mol; 19 % Ausbeute) einer farblosen Flüssigkeit.

**¹H-NMR** (CDCl₃, 400 MHz): δ = 3.63 (t, 2H; *J* = 6.6 Hz; O-CH₂), 2.67 (d, 1H; *J* = 0.8 Hz; OH), 2.22 (t, 2H; *J* = 7.4 Hz; (C=O)CH₂), 1.63 - 1.56 (m, 4H; CH₂), 1.44 (s, 9H; CH₃), 1.43 - 1.34 (m, 2H; CH₂).

**¹³C-NMR** (CDCl₃, 100.6 MHz): δ = 173.2 (C=O), 80.0 (C), 62.3 (CH₂), 35.4 (CH₂), 32.2 (CH₂), 28.0 (CH₃), 25.1 (CH₂), 24.6 (CH₂).

### 2. Stufe: 6-Methacryloyloxyhexansäure-tert.-butyleste

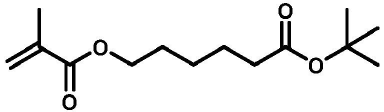

Eine Lösung von 6-Hydroxyhexansäure-*tert*.-butylester (11.15 g, 59.2 mmol), Triethylamin (7.18 g, 71.0 mmol) und *N*,*N*-Dimethylaminopyridin (0.36 g, 3.0 mmol) in Toluol (50 ml) wurde auf 0 °C abgekühlt. Eine Lösung von Methacrylsäureanhydrid (10.95 g, 71.0 mmol) in Toluol (10 ml) wurde zugetropft, und das Reaktionsgemisch wurde 1 h bei 0 °C und 1 h bei Umgebungstemperatur gerührt. Die Reaktionslösung wurde mit Salzsäure (1N, 3x 100 ml), Natronlauge (1N, 3x 100 ml), Wasser (2x 100 ml) und gesättigter wässriger Natriumchlorid-Lösung (100 ml) gewaschen, über wasserfreiem Natriumsulfat getrocknet, filtriert und am Rotationsverdampfer eingeengt. Man erhielt 14.35 g (56.0 mmol; 95 % Ausbeute) einer farblosen Flüssigkeit.

**¹H-NMR** (CDCl₃, 400 MHz): δ = 6.09 (m, 1H; =CH), 5.54 (m, 1H; =CH), 4.15 (t, 2H; *J* = 6.5 Hz; O-CH₂), 2.23 (t, 2H; *J* = 7.4 Hz; (C=O)CH₂), 1.94 (m, 3H; CH₃), 1.74 - 1.60 (m, 4H; CH₂), 1.44 (s, 9H; CH₃), 1.43 - 1.37 (m, 2H; CH₂).

**¹³C-NMR** (CDCl₃, 100.6 MHz): δ = 172.8 (C=O), 167.3 (C=O), 136.4 (=C), 125.1 (=CH₂), 79.9 (C), 64.4 (CH₂), 35.3 (CH₂), 28.2 (CH₂), 28.0 (CH₃), 25.4 (CH₂), 24.6 (CH₂), 18.2 (CH₃).

### 3. Stufe: 6-Methacryloyloxyhexansäure

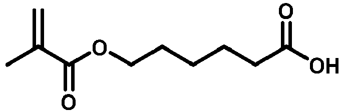

6-Methacryloyloxyhexansäure-*tert*.-butylester (8.79 g, 34.3 mmol) wurde in Dichlormethan (100 ml) gelöst, und Trifluoressigsäure (20 ml) wurde zugegeben. Das Reaktionsgemisch wurde bei Umgebungstemperatur gerührt. Nach 18 h wurde die Lösung am Rotationsverdampfer eingeengt. Trifluoressigsäure-Rückstände wurden mittels azeotroper Destillation mit Toluol entfernt, und man erhielt 6.87 g (34.3 mmol; 100 % Ausbeute) einer leicht gelblichen Flüssigkeit.

**¹H-NMR** (CDCl₃, 400 MHz): δ = 11.40 (br s, 1H; OH), 6.10 (m, 1H; =CH), 5.56 (m, 1H; =CH), 4.16 (t, 2H; *J* = 6.5 Hz; O-CH₂), 2.39 (t, 2H; *J* = 7.4 Hz; (C=O)CH₂), 1.94 (m, 3H; CH₃), 1.76 - 1.66 (m, 4H; CH₂), 1.49 - 1.41 (m, 2H; CH₂).

**¹³C-NMR** (CDCl₃, 100.6 MHz): δ = 179.7 (C=O), 167.6 (C=O), 136.2 (=C), 125.4 (=CH₂), 64.4 (CH₂), 33.7 (CH₂), 28.1 (CH₂), 25.3 (CH₂), 24.1 (CH₂), 18.1 (CH₃).

### 4. Stufe: 6-Methacryloyloxyhexansäurechlorid

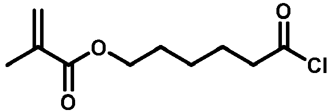

Oxalsäuredichlorid (5.15 g, 40.6 mmol) wurde zu einer Lösung von 6-Methacryloyloxyhexansäure (6.77 g, 33.8 mmol) und *N*,*N*-Dimethylformamid (0.1 ml) in Dichlormethan (100 ml) zugetropft. Die Reaktionslösung wurde 3 h bei Umgebungstemperatur gerührt und anschließend am Rotationsverdampfer eingeengt. Man erhielt 7.18 g (32.8 mmol; 97 % Ausbeute) einer gelblichen Flüssigkeit.

**¹H-NMR** (CDCl₃, 400 MHz): δ = 6.08 (m, 1H; =CH), 5.56 (m, 1H; =CH), 4.15 (t, 2H; *J* = 6.5 Hz; O-CH₂), 2.93 (t, 2H; *J* = 7.4 Hz; (C=O)CH₂), 1.94 (m, 3H; CH₃), 1.80 - 1.68 (m, 4H; CH₂), 1.50 - 1.43 (m, 2H; CH₂).

**¹³C-NMR** (CDCl₃, 100.6 MHz): δ = 173.4 (C=O), 167.2 (C=O), 136.2 (=C), 125.2 (=CH₂), 64.0 (CH₂), 46.7 (CH₂), 28.0 (CH₂), 24.7 (CH₂), 24.5 (CH₂), 18.1 (CH₃).

### 5. Stufe: N-(2-Methacryloyloxyhexanoyl)-thioharnstoff

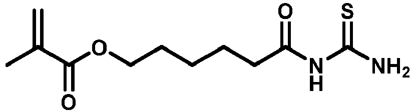

6-Methacryloyloxyhexansäurechlorid (7.05 g, 32.2 mmol) wurde in Toluol (80 ml) gelöst. Thioharnstoff (2.70 g, 35.5 mmol) wurde zugegeben, und die Suspension wurde 3 h am Rückfluss erhitzt. Nach dem Abkühlen wurde die Suspension filtriert. Der Filtrationsrückstand wurde mit Toluol (100 ml) gewaschen und getrocknet, in Wasser (100 ml) suspendiert, filtriert, mit Wasser (2x 50 ml) gewaschen und getrocknet. Der gelbliche Feststoff wurde bei 60 °C in Toluol (50 ml) gelöst. Die Lösung wurde auf 0 °C abgekühlt und kalt filtriert. Der Filtrationsrückstand wurde mit kaltem Toluol (2x 10 ml) gewaschen und getrocknet. Man erhielt 1.18 g (4.6 mmol; 14 % Ausbeute) eines weissen Feststoffs (Smp.: 97 °C).

**¹H-NMR** (CDCl₃, 400 MHz): δ = 9.95 (br s, 1H; NH), 9.61 (br s, 1H; NH), 7.54 (br s, 1H; NH), 6.10 (s, 1H; =CH), 5.56 (s, 1H; =CH), 4.16 (t, 2H; *J* = 6.5 Hz; O-CH₂), 2.41 (t, 2H; *J* = 7.4 Hz; (C=O)CH₂), 1.94 (s, 3H; CH₃), 1.77 - 1.64 (m, 4H; CH₂), 1.50 - 1.39 (m, 2H; CH₂).

**¹³C-NMR** (CDCl₃, 100.6 MHz): δ = 182.0 (C=S), 173.8 (C=O), 167.5 (C=O), 136.3 (=C), 125.4 (=CH₂), 64.2 (CH₂), 36.8 (CH₂), 28.1 (CH₂), 25.3 (CH₂), 24.1 (CH₂), 18.2 (CH₃).

### Beispiel 2

### Synthese von N-(4-Vinylbenzoyl)-thioharnstoff

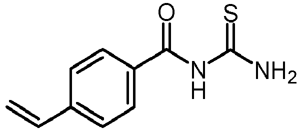

### 1. Stufe: 4-Vinylbenzoylchlorid

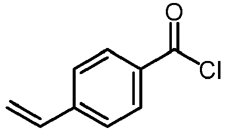

4-Vinylbenzoesäure (16.00 g, 0.108 mol) wurde mit Dichlormethan (100 ml) und N,N-Dimethylformamid (0.1 ml) versetzt und Oxalsäuredichlorid (16.45 g, 0.130 mol) wurde zugetropft. Das Reaktionsgemisch wurde 4 h bei RT gerührt und anschliessend über Kieselgel filtriert. Das Filtrat wurde am Rotationsverdampfer eingeengt, und man erhielt 16.51 g (99.0 mmol; 92%) einer leicht bräunlichen Flüssigkeit.

**¹H-NMR** (CDCl₃, 400 MHz): δ = 8.05 (d, 2H; *J* = 8.5 Hz; Ar-H), 7.50 (d, 2H; *J* = 8.4 Hz; Ar-H), 6.75 (dd, 1H; *J* = 10.9 Hz, 17.6 ; =CH), 5.93 (d, 1H; *J* = 17.6 ; =CH), 5.48 (d, 1H; *J* = 10.9 ; =CH).

**¹³C-NMR** (CDCl₃, 100.6 MHz): δ = 167.8 (C=O), 144.3 (Ar-C), 135.3 (=CH), 132.0 (Ar-C), 131.8 (Ar-CH), 126.5 (Ar-CH), 118.4 (=CH₂).

### 2. Stufe: N-(4-Vinylbenzoyl)-thioharnstoff

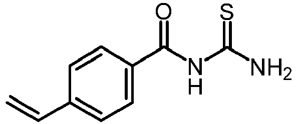

4-Vinylbenzoylchlorid (16.41 g, 98.5 mmol) wurde in Toluol (150 ml) gelöst und Thioharnstoff (8.25 g, 0.108 mol) wurde zugegeben. Die Suspension wurde 5 h am Rückfluss erhitzt und nach dem Abkühlen filtriert. Der Filtrationsrückstand wurde mit Toluol (100 ml) gewaschen, mit Wasser (300 ml) versetzt und auf 60 °C erwärmt. Nach dem Abkühlen wurde die Suspension filtriert. Der Filtrationsrückstand wurde mit Wasser (5x 100 ml) gewaschen und getrocknet. Der gelbliche Feststoff wurde mit Dichlormethan (500 ml) versetzt und am Rückfluss erhitzt. Die Suspension wurde filtriert. Das Filtrat wurde am Rotationsverdampfer eingeengt. Der gelbliche Feststoff wurde in Ethylacetat (100 ml) gelöst und über Kieselgel filtriert. Das Filtrat wurde am Rotationsverdampfer eingeengt und man erhielt 3.90 g (18.9 mmol; 19%) eines weissen Feststoffs.

**¹H-NMR** (DMSO-*d₆*, 400 MHz): δ = 11.25 (s, 1H; NH), 9.91 (s, 1H; NH), 9.60 (s, 1H; NH), 7.95 (d, 2H; *J* = 8.4 Hz; Ar-H), 7.61 (d, 2H; *J* = 8.4 Hz; Ar-H), 6.82 (dd, 1H; *J* = 10.9 Hz, 17.7 ; =CH), 6.02 (d, 1H; *J* = 17.5 ; =CH), 5.44 (d, 1H; *J* = 11.2 ; =CH).

**¹³C-NMR** (DMSO-*d₆*, 100.6 MHz): δ = 182.5 (C=S), 167.8 (C=O), 141.9 (m, Ar-C), 136.9 (m, =CH), 135.4 (m, Ar-C), 131.7 - 125.3 (m, Ar-CH), 119.4 - 116.2 (m, =CH₂).

### Beispiel 3

### N-(2-Methacryloyloxyethoxysuccinoyl)-thioharnstoff

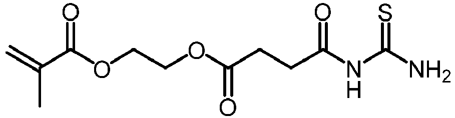

### 1. Stufe: 3-(2-Methacryloyloxyethyloxycarbonyl)propionsäurechlorid

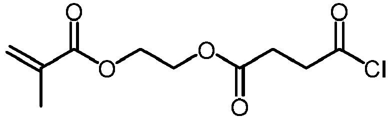

Bernsteinsäure-mono(methacryloyloxyethyl)ester (11.51 g, 50.0 mmol) wurde in Dichlormethan (80 ml) gelöst. *N*,*N*-Dimethylformamid (0.1 ml) wurde zugegeben, und Oxalsäuredichlorid (6.35 g, 50.0 mmol) wurde zugetropft. Die Reaktionslösung wurde 3 h bei Umgebungstemperatur gerührt und anschließend am Rotationsverdampfer eingeengt. Man erhielt 12.39 g (49.8 mmol; 100%) einer farblosen Flüssigkeit.

**¹H-NMR** (CDCl₃, 400 MHz): δ = 6.03 (s, 1H; =CH), 5.61 (s, 1H; =CH), 4.37 (s, 4H; OCH₂), 3.23 (t, 2H; *J* = 6.5 Hz; CH₂), 2.72 (t, 2H; *J* = 6.5 Hz; CH₂), 1.95 (s, 3H; CH₃). **¹³C-NMR** (CDCl₃, 100.6 MHz): δ = 172.8 (C=O), 170.5 (C=O), 166.9 (C=O), 135.7 (=C), 126.1 (=CH₂), 62.7 (O-CH₂), 62.0 (O-CH₂), 41.5 (CH₂), 29.1 (CH₂), 18.1 (CH₃).

### 2. Stufe: N-(2-Methacryloyloxyethoxysuccinoyl)-thiohamstoff

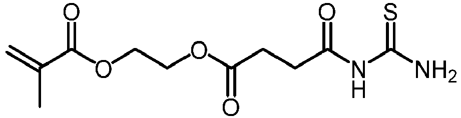

3-(2-Methacryloyloxyethyloxycarbonyl)propionsäurechlorid (12.43 g, 50.0 mmol) wurde in Toluol (100 ml) gelöst. Thioharnstoff (4.19 g, 55.0 mmol) wurde zugegeben, und die Suspension wurde 2 h am Rückfluss erhitzt. Nach dem Abkühlen wurde die Suspension filtriert. Der Filtrationsrückstand wurde mit Toluol (100 ml) gewaschen und getrocknet und man erhielt 2.44 g (8.4 mmol; 17%) eines weißen Feststoffs (Schmelzpunkt: 151 °C).

**¹H-NMR** (DMSO-*d₆*, 400 MHz): δ = 11.18 (s, 1H; NH), 9.53 (s, 1H; NH), 9.35 (s, 1H; NH), 6.03 (m, 1H; =CH), 5.69 (m, 1H; =CH), 4.28 (s, 4H; O-CH₂), 2.71-2.54 (m, 4H; CH₂), 1.88 (s, 3H; CH₃).

**¹³C-NMR** (DMSO-*d₆*, 100.6 MHz): δ = 182.0 (C=S), 173.7 (C=O), 172.4 (C=O), 166.8 (m, C=O), 135.6 (m, =C), 128.0 (m, =CH₂), 126.4 (m, =CH₂), 62.6 (m, O-CH₂), 30.0 (m, N-CH₂), 17.7 (CH₃).

### Beispiel 4 (Vergleichsbeispiel)

### Synthese von Methacrylsäure-4-oxo-9-thioxo-5-oxa-3,8,10-triazatridec-12-en-1-ylester

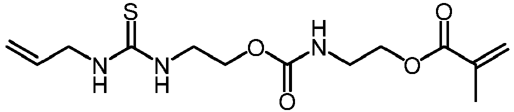

*N*-Allyl-*N*'-(2-hydroxyethyl)thioharnstoff (8.01 g, 50.0 mmol) wurde in wasserfreiem Tetrahydrofuran (80 ml) gelöst und Isocyanatoethylmethacrylat (7.76 g, 50.0 mmol) wurde zugetropft. Dibutylzinndilaureat (0.24 g, 2.0 mmol) wurde zugegeben und das Reaktionsgemisch wurde bei RT gerührt. Nach 20 h wurde die klare farblose Lösung am Rotationsverdampfer eingeengt. Das farblose Öl wurde in Ethylacetat (50 ml) gelöst und über Kieselgel filtriert. Das Filtrat wurde am Rotationsverdampfer eingeengt und man erhielt 15.45 g (48.9 mmol; 98%) eines wachsartiger weissen Feststoffs.

**¹H-NMR** (CDCl₃, 400 MHz): δ = 7.04-6.65 (m, 2H; NH), 6.13 (s, 1H; =CH), 5.92-5.80 (m, 1H; =CH), 5.62 (s, 1H; =CH), 5.60-5.49 (m, 1H; NH), 4.34-4.19 (m, 4H; CH₂), 4,19-3.64 (m, 4H; CH₂), 3.54-3.32 (m, 2H; N-CH₂), 1.95 (s, 3H; CH₃).

**¹³C-NMR** (CDCl₃, 100.6 MHz): δ = 182.0 (C=S), 167.3 (C=O), 156.7 (C=O), 135.6 (=C), 133.1 (=CH), 126.2 (=CH₂), 117.0 (=CH₂), 63.3 (m, O-CH₂), 63.2 (m, O-CH₂), 46.4 (N-CH₂), 44.4 (N-CH₂), 40.1 (N-CH₂), 18.1 (CH₃).

### Beispiel 5

### Kompositzemente auf der Basis erfindungsgemäßer Thioharnstoffe

Aus einer Mischung der Dimethacrylate UDMA (Additionsprodukt aus 2-Hydroxyethylmethacrylat und 2,2,4-Trimethylhexamethylen-1,6-diisocyanat), Bis-GMA (ein Additionsprodukt aus Methacrylsäure und Bisphenol-A-diglycidylether) und 2-(2-Biphenyloxy)-ethylmethacrylat (MA-836) sowie den Stabilisatoren MEHQ (Hydrochinonmonomethylether) und TEMPO (2,2,6,6-Tetramethylpiperidin-1-yl)oxyl), dem Füllstoff GM27884 0.7 µm sil. (silanisierter Glasfüller GM G018-056, mittlere Teilchengröße 0,7 µm, Schott) sowie den Initiatorkomponenten CHP (Cumolhydroperoxid, 80 %-ig), Kupfer(II)-acetylacetonat (Cu(acac)₂) und jeweils einer Thioharnstoff-Verbindung wurden chemisch härtenden Kompositzemente jeweils bestehend aus einer Basen-Paste und einer Katalysator-Paste hergestellt.

**Tabelle 1: Zusammensetzung der Katalysatorpaste Kat-1**

| **Komponente** | **Anteil (Gew.-%)** |
|---|---|
| Bis-GMA | 10,020 |
| UDMA | 13,352 |
| MA-836 | 10,020 |
| CHP | 1,590 |
| MEHQ | 0,018 |
| GM27884 0.7um sil. | 65,000 |
| Summe | 100,00 |

**Tabelle 2: Zusammensetzung der Base Pasten**

| **Komponente** | **Base 1*)** | **Base 2** | **Base 3** | **Base 4*)** | **Base 5*)** |
|---|---|---|---|---|---|
| Bis-GMA | 10,244 | 10,118 | 10,192 | 10,319 | 10,034 |
| UDMA | 13,663 | 13,515 | 13,613 | 13,785 | 13,414 |
| MA-836 | 10,244 | 10,118 | 10,192 | 10,319 | 10,034 |
| Hexanoylthioharnstoff | 0,820 | 0 | 0 | 0 | 0 |
| N-(2-Methacryloyloxyhexanoyl)-thioharnstoff (aus Beispiel 1) | 0 | 1,220 | 0 | 0 | 0 |
| N-(4-Vinylbenzoyl)-thioharnstoff (aus Beispiel 2) | 0 | 0 | 0,974 | 0 | 0 |
| Allylthioharnstoff | 0 | 0 | 0 | 0,548 | 0 |
| Methacrylsäure-4-oxo-9-thioxo-5-oxa-3,8,10-triazatridec-12-en-1-ylester (aus Beispiel 4) | 0 | 0 | 0 | 0 | 1,489 |
| Cu(acac)₂ | 0,004 | 0,004 | 0,004 | 0,004 | 0,004 |
| MEHQ | 0,018 | 0,018 | 0,018 | 0,018 | 0,018 |
| TEMPO | 0,007 | 0,007 | 0,007 | 0,007 | 0,007 |
| GM27884 0.7um sil. | 65,000 | 65,000 | 65,000 | 65,000 | 65,000 |
| Summe | 100,00 | 100,00 | 100,00 | 100,00 | 100,00 |

| | | | | | |
|---|---|---|---|---|---|
| *) Vergleichsbeispiel | | | | | |

Die Katalysatorpaste Kat-1 wurde mit den verschiedenen Basenpasten im Volumenverhältnis 1:1 vermischt und die Verarbeitungszeit der erhaltenen Zemente bestimmt. Die Bestimmung der Verarbeitungszeit (VZ) der Pastenmischungen erfolge mittels eines Rheometers (Motion Compact Rheometer MCR 302, Anton Paar). Hierzu wurden die Katalysatorpaste und die Basenpasten im Verhältnis 1:1 von Hand auf einem Mischblock vermengt. Anschließend wurde das Material am Rheometer auf einen aus Delrin bestehenden Stempel mit aufgerauter Oberfläche aufgetragen. Eine an einer Spindel befestigte Messkörperachse mit einer ebenfalls aufgerauten Oberfläche drückt die Probe zusammen und bestimmt bei leichter Rotation das Speichermodul. Beim Beginn der stabilen Phase sowie nach Erreichen einer bestimmten Steigung wurde jeweils ein Wendepunkt definiert. Die Wendepunkte wurden anschließend durch eine Gerade verbunden. Von dieser Geraden wurde der am weitesten Entfernte Messpunkt als VZ definiert. Die gesamte Messung wurde bei 28,7°C in einer Temperierkammer durchgeführt. Die Ergebnisse sind in Tabelle 3 angegeben.

**Tabelle 3: Verarbeitungszeiten (VZ) der Zemente**

| **Zement** | **Thioharnstoff-Derivat** | **VZ bei 28,7°C [s]** |
|---|---|---|
| Kat-1 + Base 1*) | Hexanoylthioharnstoff | 123 ± 3 |
| Kat-1 + Base 2 | N-(2-Methacryloyloxyhexanoyl)-thioharnstoff (aus Beispiel 1) | 151 ± 4 |
| Kat-1 + Base 3 | K247 N-(4-Vinylbenzoyl)-thioharnstoff (aus Beispiel 2) | 360 ± 1 |
| Kat-1 + Base 4*) | Allylthioharnstoff | 242 ± 1 |
| Kat-1 + Base 5*) | Methacrylsäure-4-oxo-9-thioxo-5-oxa-3,8,10-triazatridec-12-en-1-ylester (aus Vergleichsbeispiel 4) | 478 ± 20 |

| | | |
|---|---|---|
| *) Vergleichsbeispiel | | |

Die Bestimmung der Biegefestigkeit und des Biege-E-Moduls der Zemente erfolgte nach der Norm EN ISO-4049 (Dentistry - Polymer-based filling, restorative and luting materials). Hierzu wurde die Katalysatorpaste Kat-1 mit den verschiedenen Base-Pasten jeweils im Volumenverhältnis 1:1 vermischt und aus der Mischung normgemäße Prüfkörper hergestellt. Diese wurden durch Lagerung bei 37°C für 45 Minuten im Wärmeschrank ausgehärtet und anschließend die mechanischen Eigenschaften gemessen. Die Ergebnisse sind in Tabelle 4 zusammengestellt.

Die Ergebnisse belegen, dass die erfindungsgemäßen Kompositzemente nach der Härtung deutlich bessere mechanische Eigenschaften als Vergleichsmaterialien mit bekannten polymerisierbaren Thioharnstoffderivate aufweisen, die mit den mechanischen Eigenschaften von Zementen auf der Basis des bewährten Initiatorsystems von CHP mit Hexanoylthioharnstoff in jede Hinsicht vergleichbar sind.

**Tabelle 4: Biegefestigkeit (BF, MPa) und Biege-E-Modul (BM, MPa) der gehärteten Zemente**

| **Zement** | **Thioharnstoff-Derivat** | **BF [MPa]** | | **BM [MPa]** | |
|---|---|---|---|---|---|
| | | **TL^{a)}** | **WL^{b)}** | **TL^{a)}** | **WL^{b)}** |
| Kat-1 + Base 1*) | Hexanoylthioharnstoff | 105,8±2,0 | 97,8±8,3 | 7121 ± 309 | 7790 ± 401 |
| Kat-1 + Base 2 | N-(2-Methacryloyloxyhexano-yl)thioharnstoff (aus Beispiel 1) | 108,3±6,4 | 111,1±7,7 | 7113 ± 308 | 7793 ± 305 |
| Kat-1 + Base 3 | N-(4-Vinylbenzoyl)thioharnstoff (aus Beispiel 2) | 91,4±5,7 | 90,1±7,6 | 7151 ± 193 | 7026 ± 443 |
| Kat-1 + Base 4*) | Allylthioharnstoff | 50,4±7,9 | 50,8±3,8 | 3256 ± 560 | 1929 ± 136 |
| Kat-1 + Base 5*) | Methacrylsäure-4-oxo-9-thioxo-5-oxa-3,8,10-triazatridec-12-en-1-ylester (aus Vergleichsbeispiel 4) | 39,5±2,5 | 34,7±5,1 | 1676 ± 67 | 884 ± 81 |

| | | | | | |
|---|---|---|---|---|---|
| *) Vergleichsbeispiel ^{a)} TL: Trockenlagerung für 24 h bei Raumtemperatur ^{b)} WL: Wasserlagerung für 24 h bei 37 °C | | | | | |

## Patentansprüche

1. Radikalisch polymerisierbarer Dentalwerkstoff, der als Initiatorsystem für die radikalische Polymerisation eine Kombination aus einem Thioharnstoffderivat und einem Hydroperoxid enthält, **dadurch gekennzeichnet, dass** er ein Thioharnstoffderivat gemäß der folgenden Formel (I) enthält: 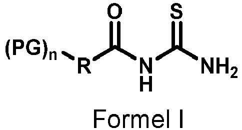 in der die Variablen die folgenden Bedeutungen haben:
R entfällt oder ein (n+1)-wertiger, aromatischer, aliphatischer, linearer oder verzweigter C₁-C₅₀-Kohlenwasserstoffrest, der durch eine oder mehrere, vorzugsweise 1 bis 8, besonders bevorzugt 1 bis 6, Ether-, Thioether-, Ester-, Amid- oder Urethangruppen unterbrochen sein kann,
PG eine radikalisch polymerisierbare (Meth)acrylat-, (Meth)acrylamid- oder Vinyl-Gruppe und
n ist 1, 2 oder 3.

2. Dentalwerkstoff nach Anspruch 1, bei dem die Variablen die folgenden Bedeutungen haben:
R ein (n+1)-wertiger, aromatischer, aliphatischer, linearer oder verzweigter C₁-C₃₀-Kohlenwasserstoffrest, der durch ein oder mehrere, vorzugsweise 1 bis 6 besonders bevorzugt 1 bis 3, Ether-, Ester- oder Urethangruppen unterbrochen sein kann,
PG eine radikalisch polymerisierbare Methacrylat-, Methacrylamid- oder Vinyl-Gruppe und
n ist 1 oder 2.

3. Dentalwerkstoff nach Anspruch 2, bei dem die Variablen die folgenden Bedeutungen haben:
R ein (n+1)-wertiger, aromatischer, aliphatischer, linearer oder verzweigter C₁-C₂₀-Kohlenwasserstoffrest, der durch 1 bis 6 Ethergruppen oder eine (1) Ester- oder Urethangruppe unterbrochen sein kann,
PG eine radikalisch polymerisierbare Methacrylat- oder Vinyl-Gruppe,
n ist 1
oder
R ein Phenylenrest, vorzugsweise eine p-Phenylenrest , oder ein Rest mit der Formel -Ph-CH₂-,
PG eine Vinyl-Gruppe und
n ist 1.

4. Dentalwerkstoff nach einem der vorrangehenden Ansprüche, der als Hydroperoxid eine Verbindung der Formel R¹-(OOH)ₘ enthält, in der R¹ ein aliphatischer oder aromatischer Kohlenwasserstoffrest und m 1 oder 2 ist.

5. Dentalwerkstoff nach Anspruch 4, der als Hydroperoxid t-Amylhydroperoxid, 1,1,3,3-Tetramethylbutylhydroperoxid, t-Butylhydroperoxid, t-Hexylhydroperoxid, 2,5-Dimethyl-2,5-di(hydroperoxy)hexan, Diisopropylbenzolmonohydroperoxid, Paramenthanhydroperoxid, p-Isopropylcumolhydroperoxid oder eine Mischung davon, vorzugsweise Cumolhydroperoxid (CHP) enthält.

6. Dentalwerkstoff nach einem der vorangehenden Ansprüche, der ein Hydroperoxid gemäß der folgenden Formel (II) enthält, 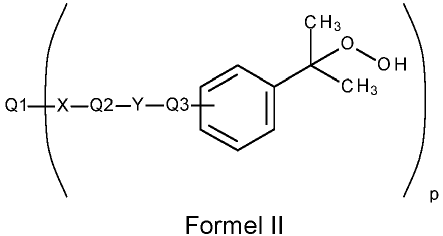 in der die Variablen die folgenden Bedeutungen haben:
Q¹ ein p-wertiger, aromatischer, aliphatischer, linearer oder verzweigter C₁-C₁₄-Kohlenwasserstoffrest, der durch ein oder mehrere S- und/oder O-Atome unterbrochen sein kann und der unsubstituiert oder durch einen oder mehrere Substituenten, die vorzugsweise aus -OH, -OR², -Cl und -Br ausgewählt sind, substituiert sein kann, wobei R² ein aliphatischer, linearer oder verzweigter C₁-C₁₀-Kohlenwassrstoffrest ist,
X, Y unabhängig voneinander jeweils entfällt, -O-, -COO-; -CONR³-, oder -O-CO-NR⁴-, wobei R³ und R⁴ unabhängig voneinander für H oder einen C₁-C₅-Alkylrest, vorzugsweise für H, Methyl und/oder Ethyl, besonders bevorzugt H stehen, und wobei X und Y vorzugsweise nicht gleichzeitig entfallen,
Q² entfällt, ein aliphatischer, linearer oder verzweigter C₁-C₁₄-Alkylen-Rest, der durch S- und/oder O-Atome unterbrochen sein kann und der unsubstituiert oder durch -OH, -OR⁵, -Cl und/oder -Br substituiert sein kann, wobei R⁵ ein aliphatischer, linearer oder verzweigter C₁-C₁₀-Kohlenwasserstoffrest ist,
Q³ eine C₁-C₃₋Alkylengruppe oder entfällt, vorzugsweise -CH₂- oder entfällt, wobei X und/oder Y entfällt, wenn Q² entfällt,
p 1, 2, 3 oder 4, und wobei
die Substitution am Aromaten in 2-, 3- oder 4-Stellung erfolgt.

7. Dentalwerkstoff nach einem der vorangehenden Ansprüche, der zusätzlich eine Übergangsmetallverbindung enthält, vorzugsweise eine Verbindung eines Übergangsmetalls, das mindestens zwei stabile Oxidationsstufen hat, vorzugsweise eine Verbindung des Kupfers, Eisens, Kobalts, Nickels, Mangans oder eine Mischung davon.

8. Dentalwerkstoff nach einem der vorangehenden Ansprüche, der
- 0,001 bis 5 Gew.-%, besonders bevorzugt 0,005 bis 3,0 Gew.-% und ganz besonders bevorzugt 0,1 bis 3,0 Gew.-% mindestens eines Thioharnstoffderivats der Formel (I).
- 0,01 bis 5 Gew.-%, bevorzugt 0,05 bis 4,0 Gew.-% und besonders bevorzugt 0,1 bis 3,0 Gew.-% Hydroperoxid,
- ggf. 0,0001 bis 1 Gew.-%, bevorzugt 0,0005 bis 0,5 Gew.-% und besonders bevorzugt 0,0007 bis 0,020 Gew.-% Übergangsmetallverbindung enthält,
jeweils bezogen auf die Gesamtmasse des Werkstoffs.

9. Dentalwerkstoff nach einem der vorangehenden Ansprüche, der zusätzlich mindestens ein radikalisch polymerisierbares Monomer enthält, vorzugsweise mindestens ein mono- oder multifunktionelles (Meth)acrylat, besonders bevorzugt mindestens ein Dimethacrylat oder eine Mischung von Mono- und Dimethacrylaten.

10. Dentalwerkstoff nach Anspruch 9, der als radikalisch polymerisierbares Monomer Methyl-, Ethyl-, 2-Hydroxyethyl-, Butyl-, Benzyl-, Tetrahydrofurfuryloder Isobornyl(meth)acrylat, p-Cumyl-phenoxyethylenglycolmethacrylat (CMP-1E), 2-(2-Biphenyloxy)-ethylmethacrylat, Bisphenol-A-dimethacrylat, Bis-GMA (ein Additionsprodukt aus Methacrylsäure und Bisphenol-A-diglycidylether), ethoxy- oder propoxyliertes Bisphenol-A-Dimethacrylat, 2-[4-(2-Methacryloyl-oxyethoxyethoxy)phenyl]-2-[4-(2-methacryloyloxyethoxy)phenyl]propan) (SR-348c), 2,2-Bis[4-(2-methacryloxypropoxy)phenyl]propan, UDMA (ein Additionsprodukt aus 2-Hydroxyethylmethacrylat und 2,2,4-Trimethylhexamethylen-1,6-diisocyanat), V-380 (ein Additionsprodukt aus einer Mischung von 0,7 mol 2-Hydroxyethylmethacrylat und 0,3 mol 2-Hydroxypropylmethacrylat mit 1 mol α,α,α',α'-Tetramethyl-m-xylylen-diisocyanat), Di-, Tri- oder Tetraethylenglycoldimethacrylat, Trimethylolpropantrimethacrylat, Pentaerythrittetramethacrylat, Glycerindi- und -trimethacrylat, 1,4-Butandioldimethacrylat, 1,10-Decandioldimethacrylat (D₃MA), Bis(methacryloyloxymethyl)tricyclo-[5.2.1.02,6]decan (DCP), ein Polyethylenglycol- oder Polypropylenglycoldimethacrylat, Polyethylenglycol-200-dimethacrylat ,Polyethylenglycol-400-dimethacrylat (PEG-200- oder PEG-400-DMA), 1,12-Dodecandioldimethacrylat oder eine Mischung davon enthält.

11. Dentalwerkstoff nach Anspruch 9 oder 10, der zusätzlich mindestens ein säuregruppenhaltiges radikalisch polymerisierbares Monomer enthält, vorzugsweise eine polymerisationsfähige Carbonsäure, Phosphonsäure, einen polymerisationsfähigen Phosphorsäureester oder ein Anhydrid dieser Stoffe.

12. Dentalwerkstoff nach einem der vorangehenden Ansprüche, der zusätzlich mindestens einen organischen oder anorganischen Füllstoff enthält, vorzugsweise ein Oxid, wie SiO₂, ZrO₂ und TiO₂ oder ein Mischoxid aus SiO₂, ZrO₂, ZnO und/oder TiO₂, einen nanopartikulären oder mikrofeinen Füllstoff, wie pyrogene Kieselsäure oder Fällungskieselsäure, Glaspulver, wie Quarz-, Glaskeramik- oder röntgenopakes Glaspulver, vorzugsweise Barium- oder Strontiumaluminiumsilikatglaspulver, einen röntgenopaken Füllstoff, wie Ytterbiumtrifluorid, Tantal(V)-oxid, Bariumsulfat, ein Mischoxid von SiO₂ mit Ytterbium(III)-oxid oder Tantal(V)-oxid, ein gemahlenes Präpolymerisat oder ein Perlpolymerisat.

13. Dentalwerkstoff nach einem der vorangehenden Ansprüche, der
(a) 0,001 bis 5,0 Gew.-%, bevorzugt 0,005 bis 3,0 Gew.-%, besonders bevorzugt 0,1 bis 3,0 Gew.-% mindestens eines Thioharnstoffderivats der Formel (I),
(b) 0,01 bis 5,0 Gew.-%, bevorzugt 0,05 bis 4,0 Gew.-% und besonders bevorzugt 0,1 bis 3,0 Gew.-% mindestens eines Hydroperoxids,
(c) 5 bis 95 Gew.-%, bevorzugt 10 bis 95 Gew.-% und besonders bevorzugt 10 bis 90 Gew.-% mindestens eines radikalisch polymerisierbaren Monomers,
(d) 0 bis 80 Gew.-% Füllstoff(e), und
(e) 0,01 bis 5 Gew.-%, bevorzugt 0,1 bis 3 Gew.-% und besonders bevorzugt 0,1 bis 2 Gew.-% Additiv(e) enthält,
jeweils bezogen auf die Gesamtmasse des Werkstoffs.

14. Dentalwerkstoff nach Anspruch 13, der 50 bis 80 Gew.-% (Füllungskomposit) oder 10 bis 70 Gew.-% (Zement) oder 0 bis 10 Gew.-% Füllstoff (Prothesenmaterial) enthält.

15. Dentalwerkstoff nach einem der vorangehenden Ansprüche zur therapeutischen Anwendung.

16. Nicht-therapeutische Verwendung eines Dentalwerkstoffs gemäß einem der Ansprüche 1 bis 14 zur extraoralen Herstellung oder Reparatur von Dentalrestaurationen, wie Prothesen, künstlichen Zähnen, Inlays, Onlays, Kronen, Brücken und Totalprothesen.

## Claims

1. Radically polymerizable dental material, which comprises a combination of a thiourea derivative and a hydroperoxide as initiator system for the radical polymerization, **characterized in that** it comprises a thiourea derivative according to the following Formula (I): 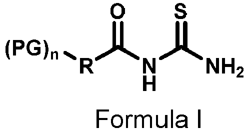 in which the variables have the following meanings:
R is absent or an (n+1)-valent, aromatic, aliphatic, linear or branched C₁-C₅₀ hydrocarbon radical, which can be interrupted by one or more, preferably 1 to 8, particularly preferably 1 to 6, ether, thioether, ester, amide or urethane groups,
PG a radically polymerizable (meth)acrylate, (meth)acrylamide or vinyl group and
n is 1, 2 or 3.

2. Dental material according to claim 1, in which the variables have the following meanings:
R an (n+1)-valent, aromatic, aliphatic, linear or branched C₁-C₃₀ hydrocarbon radical, which can be interrupted by one or more, preferably 1 to 6, particularly preferably 1 to 3, ether, ester or urethane groups,
PG a radically polymerizable methacrylate, methacrylamide or vinyl group and
n is 1 or 2.

3. Dental material according to claim 2, in which the variables have the following meanings:
R an (n+1)-valent, aromatic, aliphatic, linear or branched C₁-C₂₀ hydrocarbon radical, which can be interrupted by 1 to 6 ether groups or one (1) ester or urethane group,
PG a radically polymerizable methacrylate or vinyl group,
n is 1
or
R a phenylene radical, preferably a p-phenylene radical, or a radical with the formula -Ph-CH₂-,
PG a vinyl group and
n is 1.

4. Dental material according to any one of the preceding claims, which comprises a compound of the formula R¹-(OOH)ₘ as hydroperoxide, in which R¹ is an aliphatic or aromatic hydrocarbon radical and m is 1 or 2.

5. Dental material according to claim 4, which comprises t-amyl hydroperoxide, 1,1,3,3-tetramethylbutyl hydroperoxide, t-butyl hydroperoxide, t-hexyl hydroxyperoxide, 2,5-dimethyl-2,5-di(hydroperoxy)hexane, diisopropylbenzene monohydroperoxide, paramenthane hydroperoxide, p-isopropylcumene hydroperoxide or a mixture thereof, preferably cumene hydroperoxide (CHP), as hydroperoxide.

6. Dental material according to any one of the preceding claims, which comprises a hydroperoxide according to the following Formula (II), 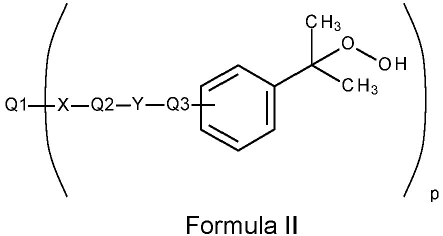 in which the variables have the following meanings:
Q¹ a p-valent, aromatic, aliphatic, linear or branched C₁-C₁₄ hydrocarbon radical, which can be interrupted by one or more S and/or O atoms and which can be unsubstituted or substituted by one or more substituents which are preferably selected from -OH, -OR², -Cl and -Br, wherein R² is an aliphatic, linear or branched C₁-C₁₀ hydrocarbon radical,
X, Y independently of each other are in each case absent, -O-, -COO-; -CONR³-, or -O-CO-NR⁴-, wherein R³ and R⁴ independently of each other represent H or a C₁-C₅ alkyl radical, preferably H, methyl and/or ethyl, particularly preferably H, and wherein X and Y are preferably not absent at the same time,
Q² is absent, an aliphatic, linear or branched C₁-C₁₄ alkylene radical, which can be interrupted by S and/or O atoms and which can be unsubstituted or substituted by -OH, -OR⁵, -Cl and/or -Br, wherein R⁵ is an aliphatic, linear or branched C₁-C₁₀ hydrocarbon radical,
Q³ a C₁-C₃ alkylene group or is absent, preferably -CH₂- or is absent, wherein X and/or Y is absent if Q² is absent,
p 1, 2, 3 or 4, and wherein
the substitution on the aromatic compound takes place in position 2, 3 or 4.

7. Dental material according to any one of the preceding claims, which additionally comprises a transition metal compound, preferably a compound of a transition metal which has at least two stable oxidation states, preferably a compound of copper, iron, cobalt, nickel, manganese or a mixture thereof.

8. Dental material according to any one of the preceding claims, which comprises
- 0.001 to 5 wt.-%, particularly preferably 0.005 to 3.0 wt.-% and quite particularly preferably 0.1 to 3.0 wt.-% of at least one thiourea derivative of Formula (I),
- 0.01 to 5 wt.-%, preferably 0.05 to 4.0 wt.-% and particularly preferably 0.1 to 3.0 wt.-% hydroperoxide,
- optionally 0.0001 to 1 wt.-%, preferably 0.0005 to 0.5 wt.-% and particularly preferably 0.0007 to 0.020 wt.-% transition metal compound,
in each case relative to the total mass of the material.

9. Dental material according to any one of the preceding claims, which additionally comprises at least one radically polymerizable monomer, preferably at least one mono- or multifunctional (meth)acrylate, particularly preferably at least one dimethacrylate or a mixture of mono- and dimethacrylates.

10. Dental material according to claim 9, which comprises methyl, ethyl, 2-hydroxyethyl, butyl, benzyl, tetrahydrofurfuryl or isobornyl (meth)acrylate, p-cumylphenoxyethylene glycol methacrylate (CMP-1E), 2-(2-biphenyloxy)ethyl methacrylate, bisphenol A dimethacrylate, bis-GMA (an addition product of methacrylic acid and bisphenol A diglycidyl ether), ethoxylated or propoxylated bisphenol A dimethacrylate, 2-[4-(2-methacryloyloxyethoxyethoxy)phenyl]-2-[4-(2-methacryloyloxyethoxy)phenyl]propane) (SR-348c), 2,2-bis[4-(2-methacryl-oxypropoxy)phenyl]propane, UDMA (an addition product of 2-hydroxyethyl methacrylate and 2,2,4-trimethylhexamethylene-1,6-diisocyanate), V-380 (an addition product of a mixture of 0.7 mol 2-hydroxyethyl methacrylate and 0.3 mol 2-hydroxypropyl methacrylate with 1 mol α,α,α',α'-tetramethyl-m-xylylene diisocyanate), di-, tri- or tetraethylene glycol dimethacrylate, trimethylolpropane trimethacrylate, pentaerythritol tetramethacrylate, glycerol di- and trimethacrylate, 1,4-butanediol dimethacrylate, 1,10-decanediol dimethacrylate (D₃MA), bis(methacryloyloxymethyl)tricyclo-[5.2.1.02,6]decane (DCP), a polyethylene glycol or polypropylene glycol dimethacrylate, polyethylene glycol 200 dimethacrylate, polyethylene glycol 400 dimethacrylate (PEG 200 DMA or PEG 400 DMA), 1,12-dodecanediol dimethacrylate or a mixture thereof, as radically polymerizable monomer.

11. Dental material according to claim 9 or 10, which additionally comprises at least one acid-group-containing radically polymerizable monomer, preferably a polymerizable carboxylic acid, phosphonic acid, a polymerizable phosphoric acid ester or an anhydride of these substances.

12. Dental material according to any one of the preceding claims, which additionally comprises at least one organic or inorganic filler, preferably an oxide, such as SiO₂, ZrO₂ and TiO₂ or a mixed oxide of SiO₂, ZrO₂, ZnO and/or TiO₂, a nanoparticulate or microfine filler, such as pyrogenic silica or precipitated silica, glass powder, such as quartz, glass ceramic or radiopaque glass powder, preferably barium or strontium aluminium silicate glass powder, a radiopaque filler, such as ytterbium trifluoride, tantalum(V) oxide, barium sulfate, a mixed oxide of SiO₂ with ytterbium(III) oxide or tantalum(V) oxide, a ground prepolymer or a pearl polymer.

13. Dental material according to any one of the preceding claims, which comprises
(a) 0.001 to 5.0 wt.-%, preferably 0.005 to 3.0 wt.-%, particularly preferably 0.1 to 3.0 wt.-% of at least one thiourea derivative of Formula (I),
(b) 0.01 to 5.0 wt.-%, preferably 0.05 to 4.0 wt.-% and particularly preferably 0.1 to 3.0 wt.-% of at least one hydroperoxide,
(c) 5 to 95 wt.-%, preferably 10 to 95 wt.-% and particularly preferably 10 to 90 wt.-% of at least one radically polymerizable monomer,
(d) 0 to 80 wt.-% filler(s), and
(e) 0.01 to 5 wt.-%, preferably 0.1 to 3 wt.-% and particularly preferably 0.1 to 2 wt.-% additive(s),
in each case relative to the total mass of the material.

14. Dental material according to claim 13, which comprises 50 to 80 wt.-% (filling composite) or 10 to 70 wt.-% (cement) or 0 to 10 wt.-% filler (prosthesis material).

15. Dental material according to one of the preceding claims for therapeutic use.

16. Non-therapeutic use of a dental material according to any one of claims 1 to 14 for the extraoral production or repair of dental restorations, such as prostheses, artificial teeth, inlays, onlays, crowns, bridges and complete dentures.

## Revendications

1. Matériau de dentisterie, polymérisable par voie radicalaire, qui contient, en tant que système amorceur pour polymérisation par voie radicalaire, une combinaison d'un dérivé de thiourée et d'un hydroperoxyde, **caractérisé en ce qu'**il contient un dérivé de thiourée de formule (I) suivante : 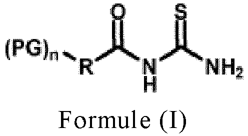 dans laquelle les symboles ont les significations suivantes :
- R ne représente rien, ou représente un reste d'hydrocarbure en C₁-C₅₀, de valence (n+1), aromatique ou aliphatique linéaire ou ramifié, qui peut être interrompu par un ou plusieurs, de préférence 1 à 8 et mieux encore 1 à 6, groupe(s) de type éther, thioéther, ester, amide ou uréthane,
- PG représente un groupe (méth)acrylate, (méth)acrylamido ou vinyle, polymérisable par voie radicalaire,
- et l'indice n vaut 1, 2 ou 3.

2. Matériau de dentisterie, conforme à la revendication 1, dans lequel les symboles ont les significations suivantes :
- R représente un reste d'hydrocarbure en C₁-C₃₀, de valence (n+1), aromatique ou aliphatique linéaire ou ramifié, qui peut être interrompu par un ou plusieurs, de préférence 1 à 6 et mieux encore 1 à 3, groupe(s) de type éther, ester ou uréthane,
- PG représente un groupe méthacrylate, méthacrylamido ou vinyle, polymérisable par voie radicalaire,
- et l'indice n vaut 1 ou 2.

3. Matériau de dentisterie, conforme à la revendication 2, dans lequel les symboles ont les significations suivantes :
- R représente un reste d'hydrocarbure en C₁-C₂₀, de valence (n+1), aromatique ou aliphatique linéaire ou ramifié, qui peut être interrompu par 1 à 6 groupe(s) de type éther ou par un (1) groupe de type ester ou uréthane,
- PG représente un groupe méthacrylate ou vinyle, polymérisable par voie radicalaire,
- et l'indice n vaut 1 ;
ou bien
- R représente un groupe phénylène, de préférence para-phénylène, ou un groupe de formule -Ph-CH₂-,
- PG représente un groupe vinyle,
- et l'indice n vaut 1.

4. Matériau de dentisterie, conforme à l'une des revendications précédentes, qui contient, en tant qu'hydroperoxyde, un composé de formule R¹-(OOH)ₘ dans laquelle R¹ représente un reste d'hydrocarbure aliphatique ou aromatique et l'indice m vaut 1 ou 2.

5. Matériau de dentisterie, conforme à la revendication 4, qui contient, en tant qu'hydroperoxyde, du tertioamyl-hydroperoxyde, du (1,1,3,3-tétraméthyl-butyl-)hydroperoxyde, du tertiobutyl-hydroperoxyde, du tertiohexyl-hydroperoxyde, du 2,5-diméthyl-2,5-di(hydroperoxy)-hexane, du monohydroperoxyde de diisopropyl-benzène, de l'hydroperoxyde de para-menthane, de l'hydroperoxyde de para-isopropylcumène, ou un mélange de ces composés, mais de préférence, de l'hydroperoxyde de cumène (HPC).

6. Matériau de dentisterie, conforme à l'une des revendications précédentes, qui contient un hydroperoxyde de formule (II) suivante : 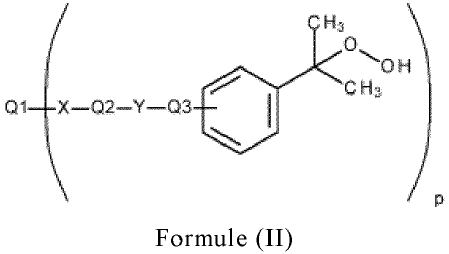 dans laquelle les symboles ont les significations suivantes :
- Q¹ représente un reste d'hydrocarbure en C₁-C₁₄, de valence p, aromatique ou aliphatique linéaire ou ramifié, qui peut être interrompu par un ou plusieurs atome(s) de soufre et/ou d'oxygène et qui peut ne porter aucun substituant ou porter un ou plusieurs substituant(s) choisi(s) de préférence parmi ceux symbolisés par -OH, -OR², -Cl ou -Br, où R² représente un reste d'hydrocarbure en C₁-C₁₀ aliphatique linéaire ou ramifié,
- chacun des symboles X et Y, indépendamment l'un de l'autre, ne représente rien ou représente un chaînon ou fragment symbolisé par -O-, -COO-, -CONR³- ou -O-CO-NR⁴- où R³ et R⁴ représentent, indépendamment l'un de l'autre, un atome d'hydrogène ou un groupe alkyle en C₁-C₅, de préférence un atome d'hydrogène ou un groupe méthyle et/ou éthyle, et en particulier des atomes d'hydrogène, étant entendu que de préférence, les symboles X et Y ne représentent pas rien simultanément,
- Q² ne représente rien ou représente un groupe alcanediyle en C₁-C₁₄ aliphatique linéaire ou ramifié, qui peut être interrompu par un ou plusieurs atome(s) de soufre et/ou d'oxygène et qui peut ne porter aucun substituant ou porter un ou des substituant(s) symbolisé(s) par -OH, -OR⁵, -Cl et/ou -Br, où R⁵ représente un reste d'hydrocarbure en C₁-C₁₀ aliphatique linéaire ou ramifié,
- Q³ représente un groupe alcanediyle en C₁-C₃ ou ne représente rien, et de préférence, représente un groupe de formule -CH₂- ou ne représente rien,
étant entendu que X et/ou Y ne représente(nt) rien, si Q² ne représente rien,
- l'indice p vaut 1, 2, 3 ou 4,
- et le ou les substituant(s) est ou sont placé(s) en position 2, 3 ou 4 sur le ou les cycle(s) aromatique(s).

7. Matériau de dentisterie conforme à l'une des revendications précédentes, qui contient en outre un composé de métal de transition, de préférence un composé d'un métal de transition possédant au moins deux états d'oxydation stables, et particulier, un composé du cuivre, du fer, du cobalt, du nickel ou du manganèse, ou un mélange de tels composés.

8. Matériau de dentisterie conforme à l'une des revendications précédentes, qui contient
- de 0,001 à 5 % en poids, de préférence de 0,005 à 3,0 % en poids, et mieux encore de 0,1 à 3,0 % en poids, d'au moins un dérivé de thiourée de formule (I),
- de 0,01 à 5 % en poids, de préférence de 0,05 à 4,0 % en poids, et mieux encore de 0,1 à 3,0 % en poids, d'hydroperoxyde,
- et en option, de 0,0001 à 1 % en poids, de préférence de 0,0005 à 0,5 % en poids, et mieux encore de 0,0007 à 0,020 % en poids, de composé de métal de transition,
chaque pourcentage étant rapporté au poids total du matériau.

9. Matériau de dentisterie conforme à l'une des revendications précédentes, qui contient en outre au moins un monomère polymérisable par voie radicalaire, de préférence au moins un acrylate ou méthacrylate monofonctionnel ou polyfonctionnel, et mieux encore, au moins un diméthacrylate ou un mélange de monométhacrylate et de diméthacrylate.

10. Matériau de dentisterie, conforme à la revendication 9, qui contient, en tant que monomère polymérisable par voie radicalaire, du (méth)acrylate de méthyle, d'éthyle, de 2-hydroxy-éthyle, de butyle, de benzyle, de tétrahydrofurfuryle ou d'isobornyle, du méthacrylate de p-cumyl-phénoxy-éthylèneglycol (CMP-1E), du méthacrylate de 2-(2-biphényloxy)-éthyle, du diméthacrylate de bisphénol A, du bis-GMA (produit d'addition d'acide méthacrylique et d'éther diglycidylique de bisphénol A), du diméthacrylate de bisphénol A éthoxylé ou propoxylé, du 2-[4-(2-méthacryloyloxy-éthoxy-éthoxy)-phényl]-2-[4-(2-méthacryloyloxy-éthoxy)-phényl]-propane (SR-348c), du 2,2-bis[4-(2-méthacryloxy-propoxy)-phényl]-propane, de l'UDMA (produit d'addition de méthacrylate de 2-hydroxy-éthyle et de 2,2,4-triméthyl-hexaméthylène-1-6-diisocyanate), du V-380 (produit d'addition d'un mélange de 0,7 mole de méthacrylate de 2-hydroxy-éthyle et de 0,3 mole de méthacrylate de 2-hydroxy-propyle avec 1 mole d'α,α,α',α'-tétraméthyl-métaxylylène-diisocyanate), du diméthacrylate de diéthylèneglycol, de triéthylèneglycol ou de tétraéthylèneglycol, du triméthacrylate de triméthylol-propane, du tétraméthacrylate de pentaérythritol, du diméthacrylate ou triméthacrylate de glycérol, du diméthacrylate de butane-1,4-diol, du diméthacrylate de décane-1,10-diol (D₃MA), du bis(méthacryloyloxyméthyl)-tricyclo[5.2.1.0^{2,6}]décane (DCP), un diméthacrylate de polyéthylèneglycol ou de polypropylèneglycol, du diméthacrylate de polyéthylèneglycol-200, du diméthacrylate de polyéthylèneglycol-400 (PEG-200-DMA ou PEG-400-DMA), du diméthacrylate de dodécane-1,12-diol, ou un mélange de ces composés.

11. Matériau de dentisterie, conforme à la revendication 9 ou 10, qui contient en outre un monomère comportant des groupes acides et polymérisable par voie radicalaire, de préférence un acide carboxylique ou phosphonique polymérisable, un ester d'acide phosphorique polymérisable, ou un anhydride de tels composés.

12. Matériau de dentisterie, conforme à l'une des revendications précédentes, qui contient en outre une charge organique ou inorganique, de préférence un oxyde tel que SiO₂, ZrO₂ et TiO₂ ou un oxyde mixte à base de SiO₂, ZrO₂, ZnO et/ou TiO₂, une charge en nanoparticules ou micro-fine, comme de la silice pyrogénée ou de la silice précipitée, une poudre de verre, comme de la poudre de quartz, de la poudre de vitrocéramique ou de la poudre de verre opaque aux rayons X, de préférence de la poudre d'aluminosilicate de baryum ou de strontium, une charge opaque aux rayons X, telle que du trifluorure d'ytterbium, de l'oxyde de tantale-(V), du sulfate de baryum, un oxyde mixte à base de SiO₂ et d'oxyde d'ytterbium-(III) ou d'oxyde de tantale-(V), un produit de prépolymérisation broyé ou un produit de polymérisation en perles.

13. Matériau de dentisterie, conforme à l'une des revendications précédentes, qui contient
a) de 0,001 à 5,0 % en poids, de préférence de 0,005 à 3,0 % en poids, et mieux encore de 0,1 à 3,0 % en poids, d'au moins un dérivé de thiourée de formule (I),
b) de 0,01 à 5,0 % en poids, de préférence de 0,05 à 4,0 % en poids, et mieux encore de 0,1 à 3,0 % en poids, d'au moins un hydroperoxyde,
c) de 5 à 95 % en poids, de préférence de 10 à 95 % en poids, et mieux encore de 10 à 90 % en poids, d'au moins un monomère polymérisable par voie radicalaire,
d) de 0 à 80 % en poids de charge(s),
e) et de 0,01 à 5 % en poids, de préférence de 0,1 à 3 % en poids, et mieux encore de 0,1 à 2 % en poids, d'adjuvant(s),
chaque pourcentage étant rapporté au poids total du matériau.

14. Matériau de dentisterie, conforme à la revendication 13, qui contient de 50 à 80 % en poids (composite pour obturation), ou de 10 à 70 % en poids (ciment), ou de 0 à 10 % en poids (matériau de prothèse), de charge.

15. Matériau de dentisterie, conforme à l'une des revendications précédentes, pour utilisation thérapeutique.

16. Utilisation non-thérapeutique d'un matériau de dentisterie, conforme à l'une des revendications 1 à 14, pour la fabrication extrabuccale ou la réparation de restaurations dentaires telles que prothèses, dents artificielles, incrustations in-lay ou on-lay, couronnes, bridges et prothèses totales.
